# EUROPEAN PATENT SPECIFICATION

(11) **EP 3 439 984 B1**
(45) Date of publication and mention of the grant of the patent: **09.02.2022**
(21) Application number: 17716009.0
(22) Date of filing: 03.04.2017
(51) Int. Cl.: B65D 75/58, B65D 1/02, B65D 47/06, B65D 51/18, B67C 3/28

(54) **TUBE CLOSURE**
ROHRVERSCHLUSS
ÉLÉMENT DE FERMETURE DE TUBE

(30) Priority: 04.04.2016 GB 201605660
(43) Date of publication of application: 13.02.2019
(73) Proprietor: Harding, Edward, Kings Lynn, Norfolk PE31 7PX (GB)
(72) Inventor: Harding, Edward, Kings Lynn, Norfolk PE31 7PX (GB)
(74) Representative: K2 IP Limited
(86) International application number: PCT/GB2017/050935
(87) International publication number: WO 2017/174971

(56) References cited:
- WO-A1-97/08096
- US-A- 4 453 653
- US-A- 5 549 226
- US-A1- 2009 179 036

## Description

The present invention is concerned with a tube closure. More specifically, the present invention is concerned with a closure for a tube for conveying fluids and fluidic solids which is arranged to be repeatedly opened and closed to selectively permit passage of a substance through the tube.

The ability to selectively close a tube, conduit or pipe is desirable in many technical fields. Some example areas include valves, spouts, liquid or gas taps, flexible pipes, pipes, tubes, openings, openings to containers, bottles, screw cap lids, lids, bags, sachets, pouches, cartons, drinking tubes or packaging which may have fluid, granules, gas or substances flowing through or stored within them.

Examples of the numerous fields in which fluid opening need to be sealed (and therefore in which the present invention could be implemented) include:
- Inflatables such as beds, toys, life vests, tents, boats and kites;
- Storage such as dry tubes and bags;
- Bottles and containers such as blow moulded disposable bottles, water bottles for sport, baby beakers, rehydration packs, paint tubes and containers;
- Valves for use in taps, in line hose connectors, safety cut-off valves, gas pipes, medical drips and flow control applications; and,
- Packaging such as spouted pouches (discussed below), sachets, cartons, food and liquid bags and bottles and box containers for "fluidic solids" such as granular substances such as cereals.

A specific example is in the field of foodstuffs. Many foods and drinks are provided in sealed containers which are opened by the end user for consumption. Baby foods are often stored in sealed foil pouches. A plastic attachment (spout) is often provided comprising a tube in fluid communication with the inside of the container. As such, the food can be squeezed or sucked out of the spout. The spout is typically sealed by a screw cap. In this way, some of the contents can be removed and the cap replaced for later use.

In some cases, the contents are consumed directly from the container rather than using a bowl or cup. Drinks are a good example of this. A flexible foil container is provided with a plastic spout having a tube which doubles as a mouthpiece or straw. The mouthpiece is sealed by a screw cap.

Conventional spouted pouches are constructed through folding, bonding and filling a laminate material and incorporating a spout. The spout includes a spout base or boat being sealed between the pouch laminates and a threaded tube, which extends from the base, which is sealed with a cap.

A problem with spouted pouches is that the production of a separate attachment, which is often twopiece (having a main part and a lid or cap) is expensive and wasteful. Even though these products are designed to be opened and resealed, they will be discarded once the contents have been consumed and as such are disposable. Therefore, there is a drive to reduce the amount of material used for both cost and environmental reasons.

Another example is a hose pipe. Users of hoses need the ability to turn the water off and on at the hose end. Until now, such solutions have been complex and expensive, involving many moving parts, which are also prone to failure. Another example is a cistern. The use of complex multi-component valve systems can make cisterns unnecessarily complex and prone to failure.

A tube closure according to the preamble of appended claim 1 is known from WO97/08096.

It is an aim of the present invention to provide an improved tube closure which can overcome, or at least mitigate, the problems discussed above.

According to a first aspect of the present invention there is provided a 1 tube closure defining a lumen (L) and comprising a valve having: a first section; a second section; and, a crease initiation region between the first and second sections, wherein: the crease initiation region formed by a narrowing of the valve and configured to initiate a crease upon relative rotation of the first and section sections; and, a protrusion projecting into the lumen (L) from the crease initiation region; in which the valve is movable between:an open condition in which there is a continuous flow path through lumen (L); and, a closed condition in which the first section is rotated relative to the second section about a crease formed in the crease initiation region feature to thereby close the lumen in the crease initiation region by abutment of the protrusion with an opposing surface of the valve.

Advantageously, the present invention offers the ability to provide a valve which may be used in the aforementioned technical fields which is both effective and inexpensive. Using a deformable tube which has crease initiation features forming a crease initiation region allows for inexpensive and repeatable results.

In order to form the crease initiation region, crease initiation features such as the following may be employed:
- A local thinning of the wall compared to at least one of the first and second sections. This reduction in bending stiffness promotes local crease formation.
- A local reduction in cross-section of the lumen therethrough. The reduction in cross section reduces the second moment of area about the axis of the crease thus promoting crease formation.
- A local reduction in the outer circumference of the tube closure. Again, the reduction in cross section reduces the second moment of area about the axis of the crease thus promoting crease formation.

According to the invention, the crease initiation region comprises a protrusion on an interior wall of the tube. This firstly limits the ability of the opposing walls of the valve to move together and form a straight line (which they are naturally attempting to do when in the buckled or folded or closed position). This increases the sealing force at the crease.

Preferably the crease initiation region defines a neck at the narrowest part of the lumen, in which the protrusion is positioned at the neck. This aligns the protrusion with the fold / crease.

Preferably the protrusion extends at least partially around the interior perimeter of the tube closurefor example along one sidewall.

More preferably the protrusion extends in an endless loop around the interior perimeter of the tube closure.

Preferably the protrusion is tapered, such that its depth of protrusion into the lumen increases with lumen width. By providing a tapered spacer between the opposing walls, both tension and compression in the X, Y and Z direction are through the seal when in a folded and closed position. The seal ridge crescent is ideally widest at the mid-point of the respective sidewall allowing a smooth transition distribute the compression evenly through the axis.

Preferably the protrusion defines a flow seal comprising a lip extending into the lumen. This further enhances the sealing effect. Preferably the flow seal bears against an interior surface of the tube in the closed condition with a sealing force, which sealing force is proportional to the pressure within the second section in the closed condition. The interior surface may be the opposing interior sidewall of the tube, or an opposing seal.

Preferably the flow seal has a first region attached to the interior sidewall of the tube, and a second, free end, which is configured to bear against the interior surface of the tube in the closed condition. Preferably the free end is configured to lie flat against the sidewall of the tube in the open condition, and movement of the first section relative to the second section moves the free end towards the opposite sidewall.

Preferably an exterior surface of the tube closure in the crease initiation region is radially inward of an interior surface of the first and / or second section in the open condition. By "radially inward" we mean further towards a central flow axis of the spout in the open condition. This promotes buckling of the closure, because a lateral force on the second section will produce an axial force component through the crease initiation region. The interruption in the load path caused by crease initiation region promotes knee-like buckling of the valve.

The first and second sections may define stiffening features extending either side of the crease initiation region. The crease initiation region is thereby defined by an interruption in the stiffening features. The stiffening features may be internal or external. The stiffening features are ribs, for example extending along the main tube flow axis. The stiffening features may also be recesses, or corrugations which resist bending in the first and second regions.

Preferably the tube closure is elongate in cross-section. In other words, it has one dimension longer than the other in a cross sectional plane perpendicular to the flow direction. This promotes crease formation along the longer axis. For example, the tube closure may be defined by two curves meeting at two-spaced apart points in cross-section. In other words, the tube closure may be lens-shaped in cross-section. The crease may extend between the two spaced-apart points.

Preferably the crease initiation region comprises two opposing sidewalls which move together when moving from the open condition to the closed condition.

Preferably, in the closed condition the first section remains open at a position spaced apart from the crease initiation region such that the first section tapers towards the crease initiation region. More preferably the first section comprises a support spaced apart from the crease initiation region, which support is configured to hold the lumen open such that in the closed condition, the first section tapers towards the crease initiation region. This is intended to cause a triangular in cross section in the closed condition (i.e. tapering from the support to the crease). This triangulation is important because it increases the force with which the sidewalls are held in contact at the crease, thereby increasing the sealing effect. It also increases the resilient force with which the tube attempts to restore its original (open) shape, which is beneficial for reopening particularly if the spout or tube is held in the closed condition and simply released to resile to the open condition.

The support may comprise a region of increased wall thickness surrounding the lumen. The support may comprise stiffening ribs. The support may also be configured to be sealed within the seal of a container, and thus provide a radially outward facing attachment and sealing surface.

Preferably the tube closure comprises a locking arrangement for releasably retaining the tube closure in the closed condition. This prevents accidental re-opening of the closure.

Preferably the locking arrangement comprises a first part on a first section side of the crease initiation region, and a second part on a second side of the crease initiation region, in which the first and second parts engage to retain the tube closure in the closed condition. This may be a clip for example.

Preferably the first part is configured to be retained in abutment with the second part in the closed condition, and in which the second part is actuable to release the first part. This facilitates "quick release".

Preferably the second part is resiliently biased into engagement with the first part, and can be released by force. Preferably the clip is spring-loaded.

The protrusion or recess is preferably on an interior side of the tube. It thereby acts as a flow seal when the tube closure is closed.

Preferably a flow seal is provided in the crease initiation region being configured to span the interior of the tube in the closed condition. The flow seal may initiate the crease.

Preferably the flow seal bears against an interior surface of the tube in the closed condition with a sealing force, which sealing force is proportional to the pressure within the second section in the closed condition.

Preferably the flow seal is flexible and extends across and covers the lumen in the closed condition such that it overlaps with the opposing sidewall or seal. This type of seal will increase sealing force with pressure in the spout in the closed condition, thus forming a self-sealing effect,

The interior surface may be the opposing interior sidewall of the tube, or may be an opposing seal.

Preferably the flow seal has a first region attached to the interior sidewall of the tube, and a second, free end, which is configured to bear against the interior surface of the tube in the closed condition. In this way the flow seal does not interrupt flow in the open condition.

Preferably the free end is configured to lie flat against the sidewall of the tube in the open condition, and movement of the first section relative to the second section moves the free end towards the opposite sidewall. This may be facilitated by a curved sidewall which moves to a flattened shape upon closure of the valve.

Preferably in the open condition the flow seal forms an endless loop on an interior side of the tube, and is configured to collapse with the crease initiation region when moving from the open to the closed condition.

Preferably there is provided means for urging the closure from the open condition to the closed condition. The means may be a tension strap configured to create a tensile force between the first section and the second section. Preferably the tension strap is resiliently biased. Preferably there is a tension strap either side of the tube.

There may also be provided means for urging the closure from the closed condition to the open condition. The means for urging may be capable of urging the spout from the closed condition to the open condition, and from the open condition to the closed condition.

Preferably a mouthpiece insert is provided inserted into at least the second section. The end of the mouthpiece insert may thereby initiate the crease. The mouthpiece insert may define a flow seal. The mouthpiece insert may be constructed from a material having a lower stiffness than the tube. This fulfils the two requirements of user comfort and seal conformability.

Preferably the tube closure is a unitary body. Preferably it is constructed from a plastics material.

According to the invention there is also provided a container comprising a tube closure according to the first aspect. Preferably the container is constructed from a flexible sheet material.

Preferably the container, comprises a separate spout assembled therewith, the spout being a tube closure according to the first aspect.

Preferably the spout is sealed within the container.

Preferably the container comprises a removable container seal, removal of which container seal along a seam exposes the spout to allow the container contents to flow through the spout.

Preferably the spout comprises a removable spout seal, which removable spout seal is connected to the container seal such that removal of the container seal removes the spout seal.

Preferably which the seam is below the crease formed in the crease initiation region.

Preferably the spout comprises spreader members depending from the spout and extending into the container, which spreader members act to hold the inside of the container open.

Preferably the spout comprises bonding members depending from the bonded to the interior of the container.

Preferably the container comprises sidewalls defining the tube closure.

Preferably there is provided at least one member attached to the exterior of the container to form at least part of the tube closure.

Preferably which the at least one member defines a crease initiation feature in the crease initiation region.

Preferably the sidewalls of the container it the region of the tube closure are reinforced.

There may be provided a tube extending from the tube closure into the container.

The disclosure also provides a method of manufacture of a tube closure according to the first aspect in which the closure is injection moulded.

There is also provided a method of manufacture of a container comprising the steps of:
providing a container;
assembling the tube closure according to the first aspect with the container.

The step of assembling the tube closure with the container may comprise the step of sealing the tube closure between laminae of the container.

The container may comprise a first sidewall and a second sidewall being attached at a seal, in which the tube closure is positioned in an interruption in the seal.

Advantageously, the present invention proposes a new form of spout which may be closed or sealed without the need of a lid or cap and which may be formed or bonded externally or internally within a sachet, pouch or other item, thus maintaining an aseptic environment and removing the need for a threaded spout. The invention allows for multiple advantages which include greatly reduced materials, more efficient simplified production methods and ease of use, improved ergonomics, compact storage and form flat weld seal production.

Various examples of the present invention will now be described with reference to the accompanying figures in which:
FIG 1A is a perspective view of a spout according to the present invention in an open, sealed condition;
FIG 1B is a perspective section view of the spout of FIG 1;
FIG 1C is a detail view of region C of FIG 2 with the spout in an open condition;
FIG 1D is a detail view of region C of FIG 2 with the spout in an closed condition;
FIG 2 is a front view of the spout of FIG 1;
FIG 3 is a side view of the spout of FIG 1;
FIG 4 is a side section view of the spout of FIG 1;
FIGs 5A to 17 are section views of various spouts according to the present invention;
FIGs 18A to 18C are a force diagrams of a spout according to the present invention;
FIGs 19A to 24C are section views of further spouts according to the invention;
FIG 25 is a perspective view of a sachet with the spout of FIG 1 assembled therewith;
FIG 26 is a perspective view of a preform incorporating a spout according to the present invention;
FIG 27 is a section view on plane B-B of the preform FIG 26;
FIG 28 is a front view of the preform of FIG 26;
FIG 29 is a section view of a moulded article moulded from the preform of FIG 26;
FIG 30 is a side view of the moulded article of FIG 29;
FIGs 31 and 32 are side section views of a cistern incorporating a valve according to the present invention;
FIGs 33 to 38 are various views of a spout in accordance with the present invention in various states;
FIGs 39A and 39B are side views of a hose pipe incorporating a valve according to the present invention;
FIG 40 is a side cross sectional view of a spout according to the present invention in a sealed condition;
FIG 41 is a side cross sectional view the spout of FIG 40 in an unsealed and open condition;
FIG 42 is a side cross sectional view of the spout of FIG 40 in a partially closed condition;
FIG 43 is a side cross sectional view of the spout of FIG 40 in a closed condition;
FIG 44 is a perspective view of a stand-up pouch assembled with a spout of the present invention in a sealed condition;
FIG 45 is a section view through a part of the pouch of FIG 44;
FIG 46 is an alternative section view to FIG 45;
FIG 47 is a perspective view of a step in the manufacture of a product incorporating a spout according to the present invention;
FIGs 48a and 48b are perspective views of a spout according to the present invention;
FIG 48c is a perspective section view of the spout of FIGs 48a and 48b;
FIG 49 is a perspective view of a spout according to the present invention;
FIGs 50a to 50c are perspective views of a spout according to the present invention; and,
FIGs 51a to 51e are perspective views of a spout according to the present invention.

In the following description of the invention, like numerals and characters designate like elements throughout the figures of the drawings.

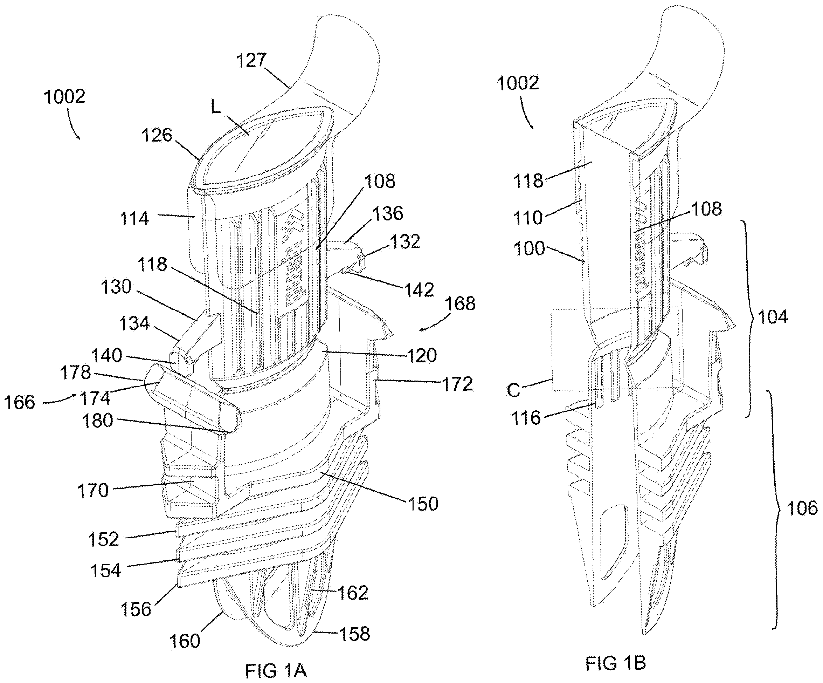

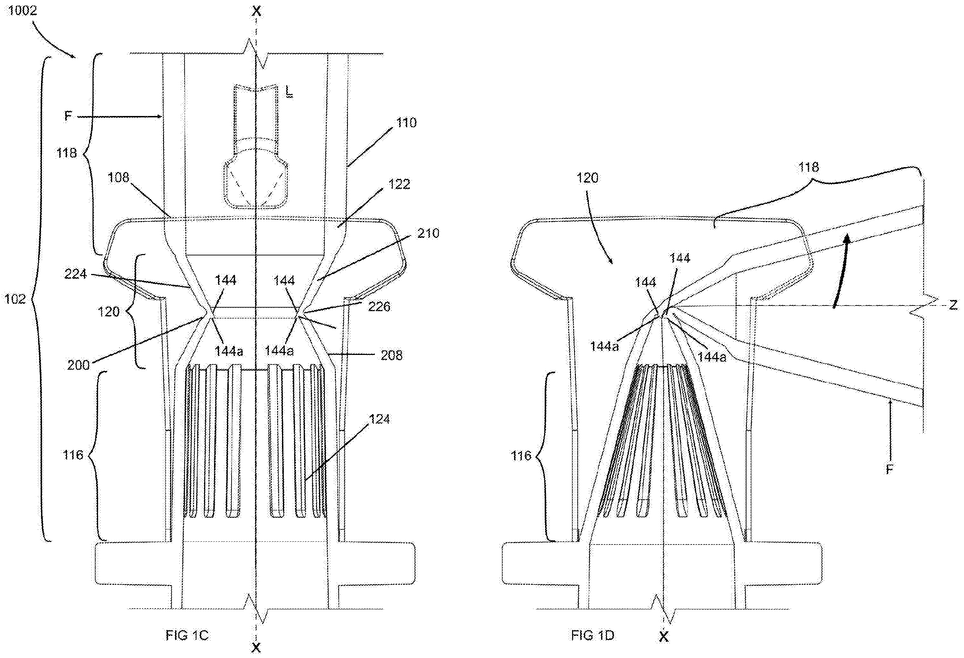

FIGs 1A to 4 are views of a spout 1002 according to the present invention. In FIGs 1 to 4, the spout 1002 is shown in an open condition.

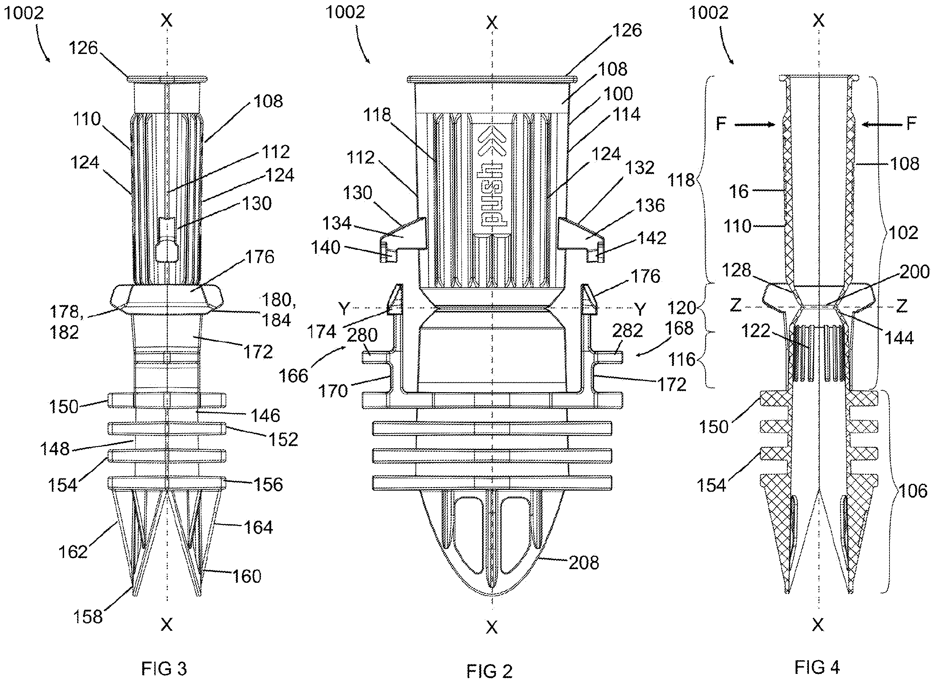

The spout 1002 comprises a unitary body 100 constructed from an injection moulded plastics material. The body 100 is generally tubular defining a lens-shaped lumen L therethrough. The spout 1002 defines a spout axis X therethrough (FIGs 3 - 5), which also defines the direction of flow of fluid through the spout 1002 when open.

The spout 1002 also defines two further axes Y and Z perpendicular to the spout axis X. The axis Y lies on the long dimension of the lens-shaped lumen (FIG 3) with the Z axis on the short dimension (FIG 4).

The spout 1002 comprises a valve 102, a locking section 104 and an attachment section 106, all of which are defined by the body 100.

The valve 102 is defined by a generally tubular part of the body 100, and is lens-shaped in cross-section having a first curved sidewall 108 and a second curved sidewall 110 which join at two spaced-apart points 112, 114. Apart from at the points 112, 114, the curved walls are spaced-apart in the Z direction to define the lumen L. In other words, in cross-section, the body encloses an area or lumen L which is a convex set bounded by two circular arcs (the curved walls 108, 110) joined to each other at their endpoints 112, 114.

Turning to FIG 4, the valve 102 comprises a first section 116 and a second section 118 defined by the curved walls 108, 110. The sections 116, 118 are spaced apart in the X direction and joined by a crease initiation region 120.

The first section 116 is adjacent the attachment section 106 and comprises a plurality of elongate ribs 122 which extend in the X direction on the interior surface of the curved walls 108, 110. The ribs 122 stop at the crease initiation region 120.

The second section 118 is on the opposite side of the crease initiation region to the first section 116 and comprises a plurality of elongate ribs 124 which extend in the X direction on the exterior surface of the curved walls 108, 110. The ribs 124 stop at the crease initiation region 120. At the free end of the second section 118 (opposite to the crease initiation region 120) there is provided a lip 126. A removable disposable seal 127 is provided which is attached to the lip 126 (shown in hidden line in FIGs 1A & 1B).

The crease initiation region 120 has smooth walls (i.e. without ribs) and has a generally thinner wall thickness than the sections 116, 118 (see Figure 4). The crease initiation region 120 also defines a recess 128 around the perimeter of the valve section 120 forming a local narrowing or neck 200 at its narrowest point between the sections 116, 118. The crease initiation region 120 is formed by the curved walls 108, 110. The wall thickness of the crease initiation region tapers (from both the first and second section 116, 118) to become thinnest at the neck 200. In other words, the crease initiation region is shaped as two frustrocones joined at their narrowest ends.

The part of the crease initiation region 120 formed by the curved wall 108 is a locally thinned and recessed section. The part of the crease initiation region 120 formed by the curved wall 110 is also locally thinned and recessed. Referring to FIG 1C, at the neck 200 there is provided a valve seal 144 extending in an endless fashion around the inner perimeter of the spout. The valve seal 144 comprises a protrusion from the walls 108, 110 into the lumen L. The protrusion comprises a depending lip 144a which extends into the lumen L. The lip 144a is tapered to a point in cross section and therefore is deformable. The valve seal 144 is therefore cantilevered and extends radially inwardly of the crease initiation region 120 (i.e. from the respective curved wall 108, 110 towards the opposite respective curved wall 110, 108). The valve seal 144 is positioned at the radially innermost point of the recessed part of the curved wall 110- i.e. at the neck 200.

The valve section 102 defines a pair of opposed retention members 130, 132 which extend from either side of the second section 118 in the Y direction (i.e. from the points 112, 114). Each retention member 130, 132 is in an inverted "L" shape having an arm 134, 136 respectively, each of which extends radially outwardly in the direction of the Y axis from the section 118. At the free end of each arm 134, 136 there is provided a tab 140, 142 respectively which is at 90 degrees to the arm 134, 136 and parallel to the X axis extending in the direction of the first section 116.

The attachment section 106 is also a generally tubular part of the body 100, but unlike the valve section 102 is inflexible (due to its generally increased wall thickness and ribs as will be described below). The attachment section 106 is lens-shaped in cross-section having a first curved sidewall 146 and a second curved sidewall 148. The curved sidewalls 146, 148 are spaced-apart in the Z direction to define part of the lumen L which is in fluid communication with the lumen in the valve section 102.

The attachment section 106 defines four annular ribs 150, 152, 154, 156 each of which is spaced-apart in the X direction and extends in the YZ plane. At the bottom-most rib 156 (furthest from the valve section 102) a pair of spreader plates 158, 160 extend in the X direction. Each spreader plate 158, 160 is generally arcuate having stiffening ribs 162, 164 respectively to hold the plate in position.

Extending from the uppermost rib 150 there is provided the locking section 104, which comprises a first flexible locking member 166 and a second flexible locking member 168. The locking members 166, 168 extend from either side of the rib 150 outwardly (i.e. in the Y direction) and upwardly (i.e. in the X direction) to form "L" shapes.

The locking members each comprise a respective locking arm 170, 172 which has a first section (attached to the rib 150) extending in the X direction, terminating in a respective tab 174, 176. The locking arms 170, 172 extend in the direction of the spout axis X to a position adjacent to (but offset from) the crease initiation region 120. Part-way along each locking arm 170, 172 there is provided a respective protrusion 280, 282 which forms a C-shaped channel with the locking arm and rib 150. This channel is suitable, and used for, machine handling of the spout 1002 when positioning in a sachet or pouch. The tabs 174, 176 extend outwardly from the arms 170, 172 to form a first pair of oppositely extending abutments 178, 180 on the arm 170 and a second pair of oppositely extending abutments 182, 184 on the arm 172. Each tab forms a "T" shape with the respective arm 170, 172 (FIG 3). As such, the rib 150 and the locking arms 170, 172 form a "U" shape which contains the crease initiation region 120 of the valve section 102. The locking arms 170, 172 are cantilevered to the rib 150 such that they can be resiliently urged towards the valve section 102 by simultaneously pressing the outside of the tabs 174, 176 as will be described below.

The abutments 178, 180, 182, 184 are positioned to be in the same Y position (i.e. in a common XZ plane) as the tabs 140, 142. The abutments 178, 180, 182, 184 cooperate with the retention members 130, 132 to provide a locking mechanism to hold the spout 1002 in the closed condition as will be described below.

In use, the spout 1002 is attached to a flexible sachet or pouch containing a liquid. A seal is formed by sealing the sachet or pouch material around the attachment section 106, and in particular to the outermost surfaces of the ribs 150, 152, 154, 156. The spreader plates 158, 160 hold the sachet or pouch walls apart to enable fluid to flow into the lumen L.

When a user wishes to remove the liquid contents, the seal 127 is removed to open the spout 1002. Some, or all of the contents can be removed.

If the user wishes to re-seal the sachet or pouch, a force F in either direction along the Z axis (FIG 4) can be applied to the second section 118 whilst holding the attachment section 106 stationary. Referring to FIGs 1C and 1D, movement from the open condition to the closed condition is shown when a force F is applied to the wall 108 in the direction of the wall 110. This creates a leverage and a torque on the valve section 102. Firstly, because the sidewall 110 in the crease initiation region is angled inwardly towards the axis X (due to the shape of the spout 1002 at this point) it does not support the vertical load arising from force F (i.e. down the sidewall 110). As such, it tends to buckle and thus collapse. Also, because the crease initiation region has a reduced second moment of area in the ZY plane due to its thinner wall thickness, smaller cross section (i.e. necked region) and lack of stiffening ribs compared to the first and second sections 116, 118 it tends to collapse. The seal ridge 144 also facilitates collapse by forming a local stress concentration. The curved walls 108, 110 flatten and move towards each other such that the seal ridges 144 are in contact (as shown in FIG 1D) and the collapsed crease initiation region 120 defines a crease axis parallel to the Y axis through the neck 200.

As the walls 108, 110 flatten and the crease axis is formed, the valve seal 144 on the curved wall 108 contacts the opposing part of the valve seal on the curved wall 110 forming a seal across the lumen L. The lips 144a meet and the force of sealing deforms them to extend downwardly along the axis X until the second section 118 starts to rotate (FIG 1D).

As the second section 118 moves towards 90 degrees of rotation as shown in FIG 1D (i.e. moves towards alignment with axis Z instead of axis X) the tabs 140, 142 of the retention members 130, 132 push the locking arms 170, 172 outwardly. As the tabs 140, 142 ride over the tabs 174, 176 of the locking arms 170, 172 the locking arms 170, 172 resile back to their original position such that the tabs 140, 142 are held under the abutments 178, 180 or 182, 184 (depending on the direction of the force F) and as such the second section 118 is prevented from resiling back into alignment with the first section 116 (and the X axis). In this condition, the spout 1002 is closed.

In the closed condition (FIG 1D), increased pressure in the first section 116 will act to compress the lips 144a against each other, thus producing a self-sealing effect.

If the user wishes to re-open the spout 1002, the first section is pulled up (force F', FIG 1D) which allows the arms 170, 172 to deform outwardly (move apart) slightly allowing passage of the tabs 140, 142 therepast. Once the tabs 140, 142 have passed, the arms 170, 172 resile to their original position. This allows the second section 118 of the valve section 102 to re-align with the first section 116. Because of the natural resilience of the crease initiation region 120, the lumen re-opens to allow the passage of fluid.

It will be noted that the attachment section 106 is not deformable. As such the lower part of the first section 116 (where it meets the attachment section 106) is held open. As such, upon collapse of the crease initiation region 120 the first section forms a triangle in cross-section (in the XZ plane). This triangular shape increases the resilience of the crease initiation region 120 to allow it to re-open when the spout is released. It also increases the sealing effect as will be described below.

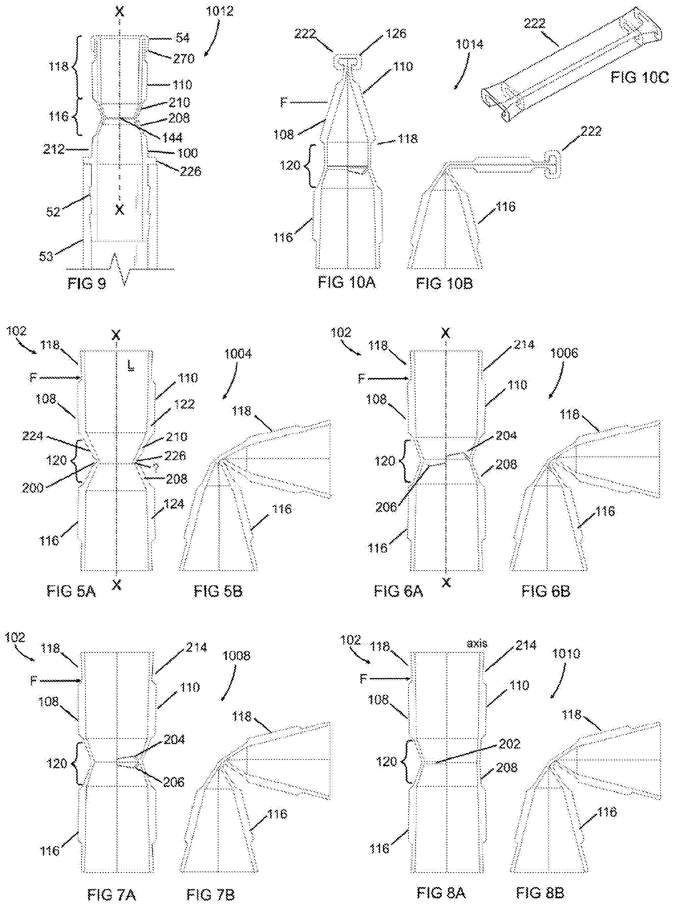

FIGS 5A to 14B are views of various spouts 1004, 1006, 1008, 1010, 1012, 1014, 1016, 1018, 1022, 1024 according to the present invention. In each case, only valve sections 102 are shown, although it will be understood that any of the valve sections 102 can be employed in the spout 1002, or used without the additional features of the spout 1002 as required.

Per the spout 1002, each valve section 102 of the spouts 1004, 1006, 1001, 1010, 1012, 1014, 1016, 1018, 1022, 1024 is lens-shaped in cross-section (per the spout 1002) having a lumen L and a main spout axis X. Each valve section comprises curved walls 108, 110 which meet at two points to define the lens-shape. The valve section 102 comprises a first section 116 (which is generally mounted or held stationary- for example when the spout is used for a sachet or pouch, the first section is generally attached thereto) and a second section 118. The sections 116, 118 are spaced along the spout axis X and have a crease initiation region 120 therebetween. The sections 116, 118 and crease initiation region 120 are constructed from the curved walls 108, 110.

Starting with FIGs 5A and 5B showing the spout 1004, the first and second sections 116, 118 have ribs 122, 124 respectively extending in the X direction on the exterior surface thereof. The crease initiation region 120 has no ribs. The parts of the curved walls 108, 110 which define the crease initiation region 120 are thinner than the parts which define the first and second sections 116, 188. The crease initiation region 120 is also recessed to form a neck 200, or a local narrowing in cross-section in the lumen L. The neck 200 is formed by two opposing V-shaped, concave (from the exterior) recesses 224, 226 in the sidewalls 108, 110 respectively.

The sidewall 110 defines a valve seal 144. The valve seal 144 extends around the curved sidewall 110 and extends both radially inwardly and downwardly in the direction of axis X towards the first section 116. The valve seal 144 is formed as a deformable lip which is cantilevered from the curved sidewall 110.

Moving from FIG 5A (the open configuration of the spout 1004) to FIG 5B (closed configuration) a force is applied in direction F. When the force F is applied to the sidewall 108 the crease initiation region 120 collapses bringing the sidewalls 108, 110 together as they flatten. Upon doing so, the valve seal 144 bears against the sidewall 108 to form a seal across the lumen L as shown in Figure 5B. An increase in pressure in the first section 116 will act to further press the valve seal 144 against the sidewall 108 to further increase the sealing effect. The seal 144 both encourages the formation of the fold or crease in the crease initiation region 102 and seals against the opposing curved wall 108, 110.

FIGs 6A and 6B show the spout 1006. The spout 1006 is similar to the spout 1004 with the exception of the crease initiation region 120.

The spout 1006 has a valve section 102 which is similar to the valve section 102 of the spout 1004. The crease initiation region 120 comprises a first valve seal 204 and a second valve seal 206. The first valve seal 204 extends around the part of the curved sidewall 110 forming the hinge section at the narrowest point of the neck 200. The first valve seal depends radially inwardly from the sidewall 110 and in also in the direction of axis X towards the second section 118. The second valve seal 206 extends around the part of the curved sidewall 108 forming the hinge section at the narrowest point of the neck 200. The second valve seal depends radially inwardly from the sidewall 110 and in also in direction X towards the first section 116 (i.e. I the opposite direction to the first valve seal 204).

When a force F is applied to the sidewall 108 the crease initiation region 120 collapses bringing the sidewalls 108, 110 together as they flatten. Upon doing so, the valve seals 204, 206 bear against the opposing sidewalls 108, 110 to form a seal across the lumen L as shown in Figure 6B. An increase in internal pressure in the first section 116 will act to further press the valve seal 206 against the sidewall 110 to further increase the sealing effect. Similarly, an increase in internal pressure in the second section 118 will act to further press the valve seal 204 against the sidewall 108 to further increase the sealing effect.

In FIGs 7A and 7B, the spout 1008 is identical to the spout 1006, with the exception that both the first and second valve seals 204, 206 extend from the part of the curved sidewall 110 defining the crease initiation region 120. The first and second valve seals 204, 206 therefore form a "V" shape facing the sidewall 108.

A force against the sidewall 108 towards sidewall 110 on the second section 118 acts to rotate the second section 118 relative to the first section 116. Both the valve seals 204, 206 bear against the sidewall 108 in the deformed crease initiation region 102 creating the desired sealing effect.

In FIGs 8A and 8B, the spout 1010 is similar to the spout 1004, although the seal 144 is not provided. Only a ridge 202 is provided on the wall 108 in the crease initiation region 120 at the neck 200. The ridge helps to initiate the crease and provides a seal within the folded spout 1010.

FIG 9 show a further embodiment of a spout 1012 assembled with a drinking straw 53.

The spout 1012 is a two-part assembly of a spout body 100 and a mouthpiece 54. At the free end of the first section 116, the spout body 100 defines a ridged mating formation 52 which is configured to mate with the straw 53 and secure the spout 1012 therewith. A flange 266 is provided to limit insertion into the straw 53.

The mouthpiece 54, is manufactured from a more flexible or rubberised material than the spout body 100. The mouthpiece 54 is a tubular body which is configured to fit into the lumen of the second section 118 of the spout body 100 and to be in fluid communication therewith. The mouthpiece comprises a valve ridge 144 at a first end. The mouthpiece 54 further comprises a return flange 270 at a second end.

The mouthpiece 54 is inserted into the spout body 154 during manufacture such that the valve seals 144 lie in the crease initiation region 120 (i.e. aligned with the local thinning of the sidewalls 108, 110).

It will be noted that as the spout 1012 is moved to a closed position, the opposing parts of the valve ridge 144 interact to form a seal, and an increase in pressure in the first section 116 will increase the sealing force as the seals 144 bear against each other.

The use of an insert 54 is beneficial, as it simultaneously provides two structures which require similar characteristics- specifically the soft, comfortable mouthpiece and the soft deformable seal ridges. This allows the spout body 100 to be constructed from stiffer, more resilient material which is beneficial for resilience.

Turning to Figures 10A to 10C, the spout 1014 sidewalls 108, 110 can be sealed together at the open end of the second section 118. The open end of the section 118 defined a radially outwardly protruding lip 126. A clasp 222 is provided which is generally "U" shaped having opposing return flanges. The clasp 222 can be slid over the lip 126 to seal the sidewalls 108, 110 together for additional sealing.

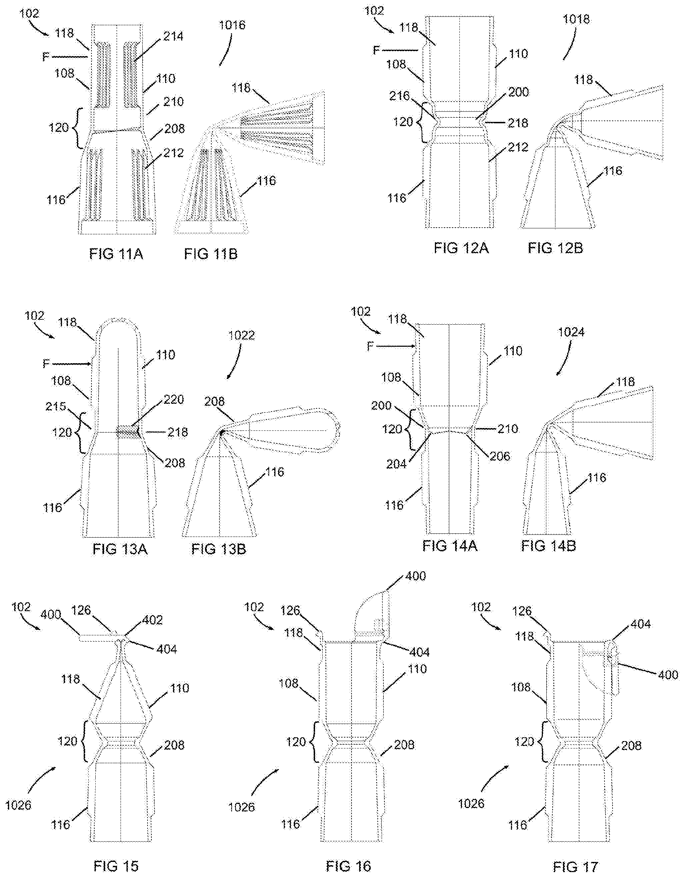

Turning to FIGs 11A and 11B, the spout 1016 comprises a valve section 102 in which the first section 116 has a larger cross-section than the second section 118 (in the previous embodiments, the valve sections 116, 118 have had the same cross-section). Both valve sections 116, 118 are lens shaped. The crease initiation region 120 comprises a frustroconical section 208 tapering inwardly from the first section 116 followed by a straight-walled section 210 which joins to the second section 118.

Both the first section 116 and the second section 118 have internal ribs 212, 214 respectively which extend along the X axis (FIGs 11A and 11B are cross sections). The ribs 212, 214 are not present in the crease initiation region 120. As such, a force F on the second section tends to collapse the weaker hinge section at the point where the sections 208, 210 meet thus sealing the spout 1016.

FIGs 12A and 12B, the spout 1018 defines a crease initiation region 120 having a neck 200 defined by a pair of semi-circular formations 216, 218 in the sidewalls 108, 110 thus forming semi-circular recesses on the exterior of the valve section 102. This further encourages the formation of the hinge in the crease initiation region 120 because the sidewalls are less able to take a load in the direction of axis X without buckling.

FIGs 13A and 13B show the spout 1022 which is similar to the spout 1016. The crease initiation region 120 comprises a separate seal component 220 attached to the sidewall 110 and extending all the way therealong. The seal component 220 is a body of material being softer (less stiff) than the material of the valve section 102 and therefore suitable for conforming to the more rigid material of the sidewall 108 when the crease initiation region collapses (FIG 13B). The spout 1022 further comprises a domed, flexible, free end 126 having a plurality of through bores 126a. The bores are formed within the dome through which fluid might be sucked or sprayed.

FIGs 14A and 14B show the spout 1024 in which the first section 116 is of lesser cross-sectional area than the second section 118. The crease initiation region 120 comprises a frustroconical section 208 tapering inwardly from the second section 118 followed by a straight-walled section 210 which joins to the first section 116. Two valve seals 204, 206 are provided at the neck 200 which extend radially inwardly and in the direction of the X axis towards the first section 116. The valve seals 204, 206 are mirror images of each other and allow folding in either direction (depending on the direction of the force F).

FIGs 15 to 17 demonstrate a spout 1026 having a lid seal which allows the top of the spout to be squeezed together and clipped shut. The spout 1026 is similar to the spout 1018 with the exception of the free end of the second section 118. A lip 126 is provided on the wall 108, and a lid 400 provided on the opposing wall 110. The lid 400 is deformable and can adopt the curvature of the wall 110. The lid 400 is connected to the wall 110 via a live hinge 404 and defines a bore 402 proximate the live hinge. The bore 402 is suitable for receiving and retaining the lip 126.

Referring to FIG 15, the walls 108, 110 at the free end of the second section 118 are flattened and adjacent, with the lip 126 engaged in, and retained by, the bore 404 in the lid 400. In FIG 16, the walls 108, 110 are apart (the open condition) and the lid 400 has adopted the curvature of the wall 110. Should the user wish to stow the lid 400, it can be flipped to the exterior side of the second section 118 (FIG 17) where its curvature matches the wall 110 and retains it in the stowed position. The curvature of the wall and lid 400 keeps it in the stowed position until the wall 110 is flattened.

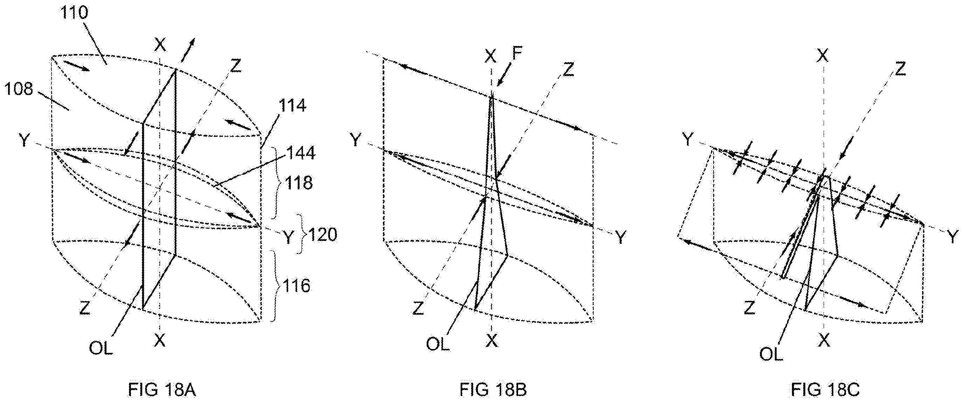

FIGs 18A to 18C show how the sealing effect of the present invention comes about. In particular, the effect is described with respect to the spout 1002, although the skilled addressee will understand that the principles apply to the other spouts described above. It will also be noted that the drawings 18A to 18C concentrate on the effect of the valve seal 144, and as such are simplified to enhance this (for example, the local narrowing of the crease initiation region 120 is not shown for clarity).

FIGs 18A to 18C show the valve section 102 comprising the first section 116, the second section 118 and the crease initiation region 120 therebetween. The crease initiation region 120 comprises the valve seal 114 extending from both sidewalls 108, 110. It will be noted that the valve seal 144 increases in thickness (i.e. in its depth of protrusion) from the ends 112, 114 towards the centre points of the walls 108, 110. Therefore, it is generally crescent-shaped on each sidewall 108, 100.

The valve section 102 is defined by opposing sidewalls 108, 110. The axes X, Y, Z are shown. An outline OL of the valve section 102 is shown in bold on the XZ plane.

FIG 18A shows the spout 1002 in the open condition with fluid able to flow through the lumen L. Because the walls 108, 110 are injection moulded in a curved form, they are dimensionally stable and hold the lumen L open.

In FIG 18B, it is assumed that the bottom end of the first section 116 is held (in particular by the user as he / she holds the pouch, sachet, hose, tube etc.). Upon application of a force in direction F (i.e. acting on the wall 110 towards the wall 108) the walls 108, 110 will start to flatten and meet (as demonstrated by the outline OL). It will be noted that the lumen is held open at the lower end of the first section 116 and as such the spout 1002 starts to form a triangle, with the lumen narrowing to a point where the walls 108, 110 meet.

Continued application of the force F encourages the walls 108, 110 to approach each other at the crease initiation region 120 where the opposite parts of the valve seal 144 abut. As this occurs, the second section (now flattened) rotated about the Y axis over the first section 116 (FIG 18C).

The seal produced by this fold is enhanced in a number of ways.

Firstly, the wall 110 must stretch over the wall 108 (i.e. it must describe a longer arc) to make the fold. This means that its natural elastic resilience in tension will tend to urge it towards the wall 108 (radially inwardly), thus increasing sealing. The presence of the valve seal 144 enhances this effect as the valve seal 144 increases the gap between the walls 108, 110 (they cannot meet at the axis Y). Therefore the stretch of the wall 110 must be even greater, and the sealing effect increased. It will be noted that because the valve seal 144 is crescent-shaped, the maximum effect is seen at the centres of the sidewalls (i.e. at the widest part of the lumen when open). This is desirable because the inherent mechanical stiffness of the walls 108, 110 is less at the centre (being furthest from the end points 112, 114). Therefore it is desirable that more sealing enhancement occurs at this position. This is demonstrated in FIG 18C where the walls 108, 100 are further apart at the centre (axis Z) than the ends even when folded. In other words, the protrusions themselves create a closed, lens-shaped cross section when the crease is initiated.

Similarly, the wall 108 feels a compressive force and tends to produce a force acting radially outwardly towards the wall 110. The inside of the arc (sidewall 108) is under compression and is attempting to travel a shorter distance therefore it acts as a knuckle pushing up into the axis Y and applying compression to the valve seal 144.

These forces tend to push the two opposing sections of the valve seal 144 together, which increases the sealing and ensures that the lumen L is tightly closed.

It will be noted that the crescent-shape will be effective for any type of internal protrusion into the lumen to enhance the sealing effect.

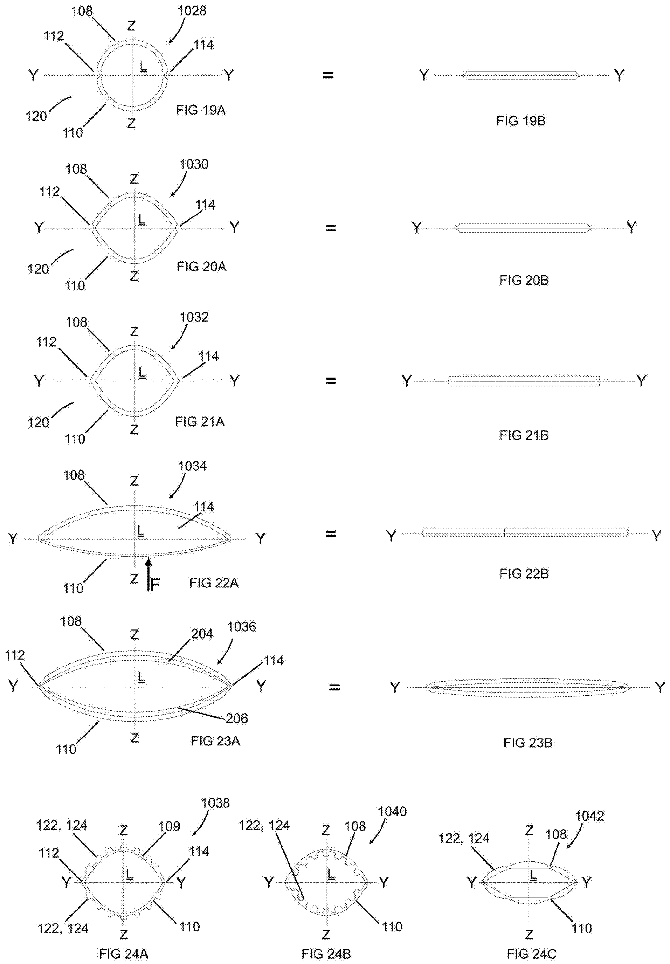

FIGs 19A to 23B show various embodiments of spouts 1028, 1030, 1032, 1034, 1036. FIGS 19A, 20A, 21A, 22A show section views through the crease initiation regions 120 of the spouts in the open condition showing the position of the sidewalls 108, 110 which meet at points 112, 114. FIGS 19B, 20B, 21B, 22B, show section views through the crease initiation regions 120 of the spouts in the closed condition showing the position of the sidewalls 108, 110.

In FIGs 19A and 19B, the sidewalls 108, 110 of the spout 1028 are each semicircular (i.e. 180 degree circle segments) forming a circular cross-section lumen L. External notches are provided at the points 112, 114 where the sidewalls meet, which encourage collapse in the Z direction to align the sidewalls on the Y axis. Upon collapse (by the same mechanism as for the earlier described embodiments) the sidewalls 108, 110 flatten, as shown in FIG 19B to seal against each other and close the lumen L.

In FIGs 20A and 20B, the sidewalls 108, 110 of the spout 1030 are each circle segments of less than 180 degrees forming a lens-shaped cross-section lumen L (per the spout 1002). External notches are provided at the points 112, 114 where the sidewalls meet, which encourage collapse in the Z direction to align the sidewalls to the Y axis. Upon collapse the sidewalls 108, 110 flatten, as shown in FIG 20B to seal against each other and close the lumen L. Because the sidewalls 108, 110 are flatter than the sidewalls of the spout 1028 the collapse comes about more easily.

In FIGs 21A and 21B, the spout 1032 is similar to the spout 1030 (i.e. it has a lens-shaped lumen L). The main exception is that no notches are provided at points 112, 114 which makes the crease initiation region 120 stiffer and resists collapse more.

In the spout 1034 of FIGs 22A and 22B, the sidewall 110 is of a greater radius of curvature (shallower) than the sidewall 108. In other words, the sidewall 108 is longer than the sidewall 110 (although they meet at points 112, 114). This enhances the sealing effect upon collapse of the hinge section. When a force F is applied to the sidewall 110 of the first section (on a first side of the hinge section as described with previous embodiments) towards the sidewall 108, the hinge section collapses. As it does so, the sidewall 110 experiences higher strain in axis Y than the sidewall 108 (as it must be stretched to meet the expanding endpoints of the sidewall 108). This causes a beneficial sealing effect by creating a tensioned seal between the sidewalls 108, 110. The sidewall 110 is made thinner to ensure that it deforms rather than the sidewall 108.

In the spout 1036 of FIGs 23A and 23B, the sidewalls 108, 110 are provided with internal seal ridges 204, 206. The seal ridges 204, 206 are of variable thickness and are crescent-shaped, thickening away from the points 112, 114 (i.e. towards their thickest point at the centre of the walls 108, 110 where the lumen L is widest in the open condition). This increases the sealing effect where it is most neededat the centre of the lumen L. Because the ridges 204, 206 resist the collapse of the spout, the sealing force is increased, making leakage less likely.

FIGS 24A to 24C show three types of surface feature which can be incorporated into the spouts 1038, 1040, 1042 in the first and / or second regions 116, 118.In each case the lens-shaped lumen L is defined by sidewalls 108, 110.

In FIG 24A (which is a cross sectional view in plane YZ of the first region 116 in FIG. 2) the walls 108, 110 have external ribs 122, 124. The ribs extend outwardly from the walls 108, 110 not radially, but in the Z direction. This allows the part to be more easily demoulded from the tool when moulding.

In FIG 24B (which is a cross sectional view in plane YZ of the first region 118 in FIG. 2) the walls 108, 110 have internal ribs 212, 214.

In FIG 24C, the walls 108, 110 (which do not relate to a previous embodiment) have wide ribs or plates and external recesses 122, 124.

In each of FIGs 24A to 24C, the main purpose of the ribs and/or recesses is to create a structure resistant to bending or buckling when the closing force F is applied. That said, each spout 1038, 1040, 1042 is made collapsible when compressed equally from both sides (flattening in the Z axis) for storage and transport. The recesses or gaps between ridges act as multiple conjoined live hinges therefore allowing material deformation and movement of the structure to be controlled.

The ribs and/or recesses also provide a method of forming a "material efficient" structure by removing a high quantity of material whilst creating an item which can be moulded rapidly due to the thinner overall wall sections (which can therefore cool quicker in the mould). As such the spouts can be manufactured faster than a conventional solid walled structure.

As can be understood from the above embodiments, the resistance of the crease initiation region 120 to collapse can be tailored to suit the application, some of which will be described below.

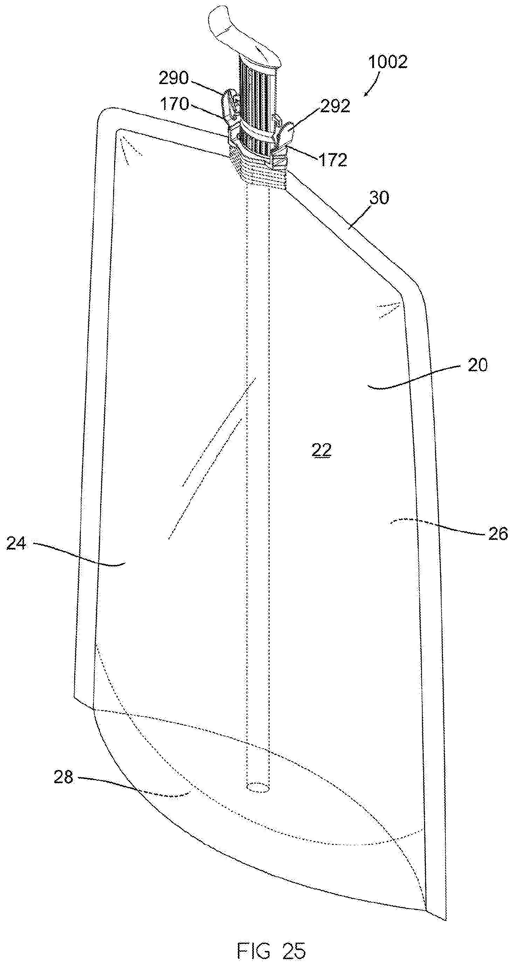

FIG 25 shows a free-standing sachet 20 defining an enclosed space 22 for liquid contents. The sachet 20 defines a first side panel 24, a second side panel 26 and a base 28. The panels 24, 26 and the base 28 are attached by a seal 30. The spout 1002 is attached to the sachet 20 such that the lumen L is in fluid communication with the space 22. This is achieved by securing the attachment section 106 between the panels 24, 26 in an interruption in the seal 30. The spout 1002 can be operated as described above to access the contents in the space 22, with the exception that the locking arms 170, 172 on the spout 1002 in FIG 25 have push tabs 290, 292 which allow the arms to be pushed inwardly to release the second section of the spout for opening. This is an alternative method to pulling the spout open.

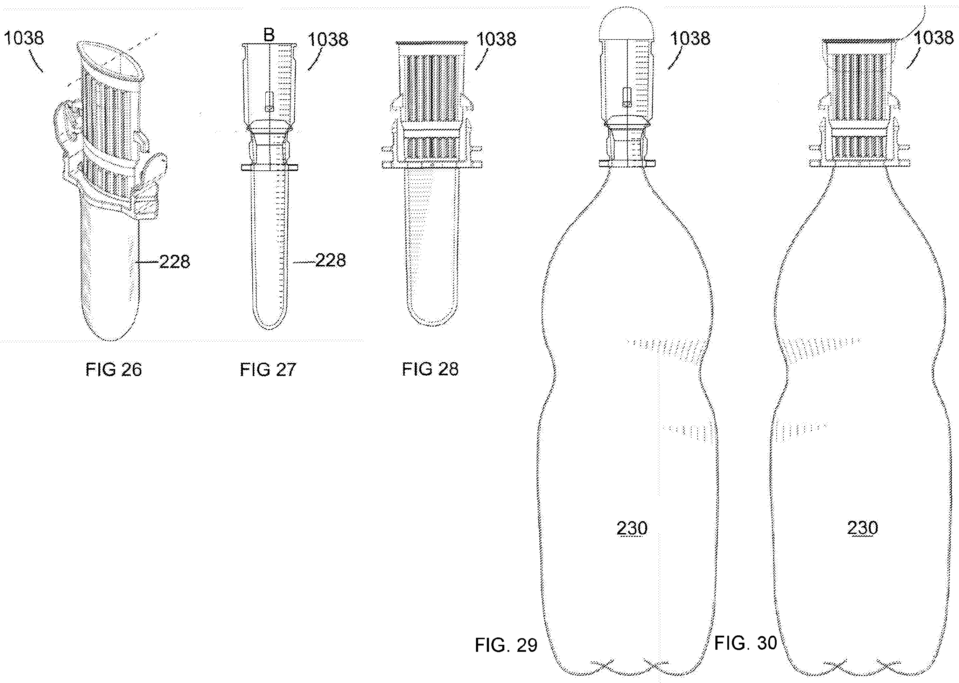

FIGs 26 to 28 show a parison for moulding a bottle which incorporates a spout 1038 according to the present invention. The spout 1038 is identical to the spout 1001, with the exception that the attachment section 106 below the rib 150 has been replaced by a bottle preform 228. The bottle preform 228 and the spout 1038 are co-moulded form a unitary component.

The preform 228 can be blow moulded in a known process to form a bottle 230 as shown in FIGs 29 and 30 having an integral spout 1038 which functions in the same way as the spout 1002. The lumen of the spout 1038 is in fluid communication with the inside of the bottle 230 and is configured to be selectively opened and closed as described above.

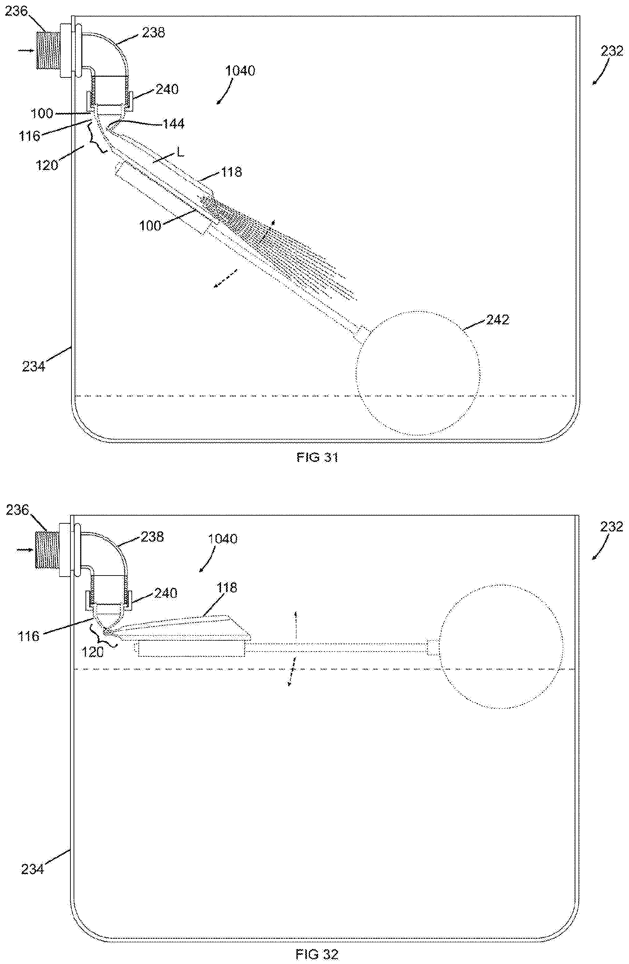

Turning to FIGs 31 and 32, a cistern 232 is shown comprising a water tank 234 and a water inlet 236. The water inlet 236 is positioned at the top of the tank 234 in a sidewall thereof, and is connected to an elbow 238 terminating in an outlet 240 directed towards the bottom of the tank (i.e. vertically downwards in use).

A valve 1040 is attached to the outlet 240. The valve 1040 comprises a unitary flexible moulded plastics body 100. The body 100 is generally tubular comprising a lumen L therethrough in communication with the outlet 240.

The valve comprises a first section 116 and a second section 118, the sections 116, 118 being connected by a crease initiation region 120. The first section 116 is attached to the outlet 240 such that it is static. The second section 118 is movable relative to the first section to open and close the lumen by selectively collapsing the crease initiation region 120 (as described with respect to the previous spout embodiments). The crease initiation region 120 defines a valve seal 144 projecting radially inwardly therefrom to seal against the opposing sidewall when the valve is closed.

The second section has a float 242 attached thereto, which can rise dependent on the water level in the tank 234. In Figure 31, the water level is low, and as such the float is low. In this condition, the second section 118 is at an angle with respect to the first section 116 such that the lumen in the crease initiation region 120 is open. As such, water can flow through the lumen L to fill the tank 234. As the float rises, the angle of the second section moves towards 90 degrees with respect to the first section 116 thus closing the crease initiation region 120. This stops the flow of water as the valve seal extends across the lumen L.

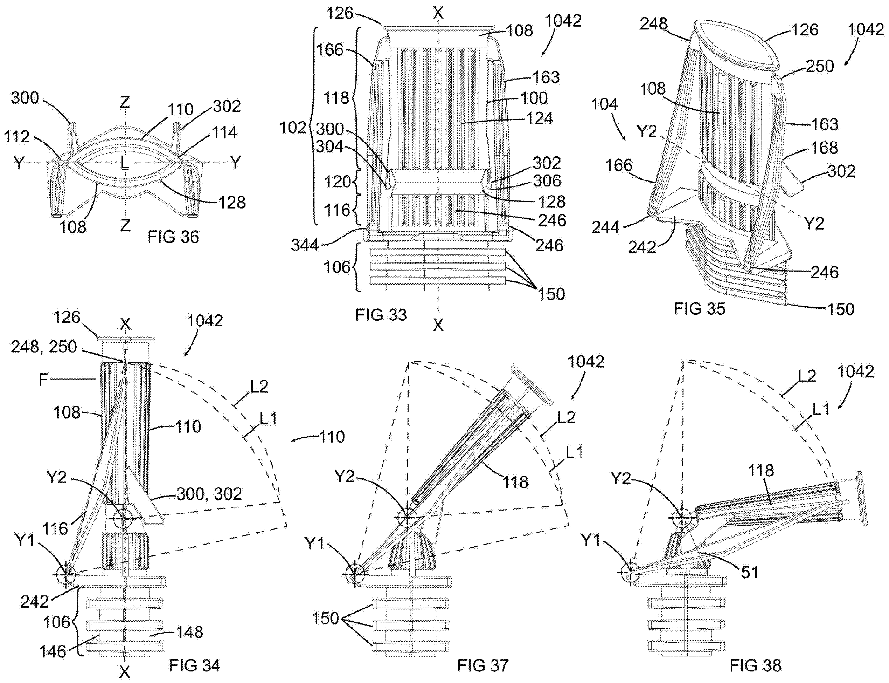

Turning to FIGs 33 to 38 there is provided a spout 1042 which can be installed in e.g. a pouch or sachet. In FIGs 33 to 36, the spout 1042 is shown in an open condition. In FIG 37 the spout 1042 is shown moving to a closed condition.

The spout 1042 comprises a unitary body 100 constructed from an injection moulded plastics material. The body 100 is generally tubular defining a lens-shaped lumen L therethrough. The spout 1042 defines a spout axis X therethrough, which also defines the direction of flow of fluid through the spout 1042 when open.

The spout 1042 also defines two further axes Y and Z perpendicular to the spout axis X. The axis Y corresponds to the larger dimension of the lumen L, and the Z axis to the shorter dimension.

The spout 1042 comprises a valve section 102, a locking section 104 and an attachment section 106, all of which are defined by the body 100.

The valve section 102 is defined by a generally tubular part of the body 100, and is lens-shaped in cross-section having a first curved sidewall 108 and a second curved sidewall 110 which join at two spaced-apart points 112, 114. Apart from at the points 112, 114, the curved walls are spaced-apart in the Z direction to define the lumen L. In other words, in cross-section, the body encloses an area or lumen L which is a convex set bounded by two circular arcs (the curved walls 108, 110) joined to each other at their endpoints.

The valve 102 comprises a first section 116 and a second section 118 defined by the curved walls 108, 110. The sections 116, 118 are spaced apart in the X direction and joined by a crease initiation region 120 (FIG 33).

The first section 116 is adjacent the attachment section 106 and comprises a plurality of elongate ribs 122 which extend in the X direction on the exterior surface of the curved walls 108, 110. The ribs 122 stop at the crease initiation region 120.

The second section 118 is on the opposite side of the crease initiation region to the first section 116 and also comprises a plurality of elongate ribs 124 which extend in the X direction on the exterior surface of the curved walls 108, 110. The ribs 124 also stop at the crease initiation region 120. At the free end of the second section 118 (opposite to the crease initiation region 120) there is provided a lip 126. A removable disposable seal (not visible) is provided which is attached to the lip 126.

The crease initiation region 120 has smooth walls and has a generally thinner wall thickness than the sections 116, 118 (see Figure 6). The crease initiation region also defines a recess 128 around the perimeter of the valve section 120 forming a necked region. The crease initiation region 120 is also formed by the curved walls 108, 100, and is locally of thinner wall thickness than the sections 116, 118.

The attachment section 106 is also generally a tubular part of the body 100, but unlike the valve section 102 is inflexible (due to its generally increased wall thickness and ribs as will be described below). The attachment section 106 is lens-shaped in cross-section having a first curved sidewall 146 and a second curved sidewall 148. The curved sidewalls 146, 148 are spaced-apart in the Z direction (other than where they join) to define part of the lumen L which is in fluid communication with the lumen in the valve section 102.

The attachment section 106 defines a plurality of annular ribs 150 each of which is spaced-apart in the X direction and extends in the YZ plane.

Extending from the uppermost rib 150 to the second section 118 there is provided the locking section 104, which comprises a first flexible locking member 166 and a second flexible locking member 168. The uppermost rib 150 defines a flange 242 which extends beyond the side of the sidewall 108 opposite the sidewall 110. The flange 242 is parallel to the YZ plane. At its furthest point, the flange is connected to each of the locking members 166, 168 by respective live hinges 244, 246. The live hinges, although spaced apart in the Y direction, are rotatable about a common axis Y1. The common axis Y1 is offset in both the X and Z directions with respect to a crease axis Y2 of the crease initiation region 120. The locking members 166, 168 are straight, inclined members which extend from each hinge 244, 246 to join the second section 118 at either side thereof at attachments 248, 250, spaced apart from the crease initiation region 120.

The locking section 104 further comprises two locking hooks 300, 302 which extend from either side of the second section 118 both downwardly and rearwardly away from the valve 102 on the same side as the wall 110 (i.e. on an opposite side to the locking members 166, 168). The hooks 300, 302 are cantilever from the second section 118. Each hook 300, 302 is generally planar (in the XZ plane) comprising an abutment 304, 306 respectively.

In use, the spout 1042 is attached to a flexible sachet or pouch containing a liquid. A seal is formed by sealing the sachet or pouch material around the attachment section 106, and in particular to the outermost surfaces of the ribs 150.

Some, or all of the contents can be removed when the spout 1042 is open. If the user wishes to re-seal the sachet or pouch, a force F in the Z direction (FIG 34) can be applied to the second section 118. This creates a torque about the crease initiation region 120. Because the crease initiation region has a reduced second moment of area in the ZY plane due to its thinner wall thickness, smaller cross section and lack of stiffening ribs compared to the first and second sections 116, 118 it collapses. The curved walls 108, 110 flatten and move towards each other to be in contact, and the collapsed crease initiation region 120 defines the crease axis Y2 parallel to the Y axis through the narrowest point of the crease initiation region 120.

Referring to FIG 34, the locus of movement L2 of the attachments 248, 250 about the crease axis Y2 is shown. This is a circular arc about the crease axis Y2. Also in FIG 34, the desired locus L1 of the attachment points 248, 250 about the common axis Y1 is shown. It will be noted that because the axis Y1 is further than the axis Y2 from the locus, the path is of a larger radius (i.e. is shallower). As such, in order to move to the position of FIG 37, the body of the spout 1042 and the locking members 166, 168 need to be deformed. Once the second section passes over 45 degrees (FIG 37), the body of the spout 1042 and the locking members 166, 168 will start to resile and pull the section 118 to the closed position (FIG 38). Once the second section 118 has reached 90 degrees rotation relative to the first section (FIG 38), the hooks 300, 302 engage the outer edges of the first section 116 and hold the second section 118 in the closed position.

If the user wishes to re-open the spout 1042, a force opposite to force F must be applied to deform and release the hooks 300, 302. The second section 118 must be moved past the 45 degree angle shown in FIG 37 to stretch the locking members 166, 168 . After this (moving back to FIG 34) the locking members 166, 168 will result and return the spout 1042 to the open condition.

It will be noted that the attachment section 106 is not deformable. As such the lower part of the first section 116 (where it meets the attachment section 106) is held open. As such, upon collapse of the crease initiation region 120 the first section forms a triangle 51 in cross-section (in the XZ plane) as shown in FIG 38. This triangular shape increases the resilience of the crease initiation region 120 to allow it to re-open when the spout is released.

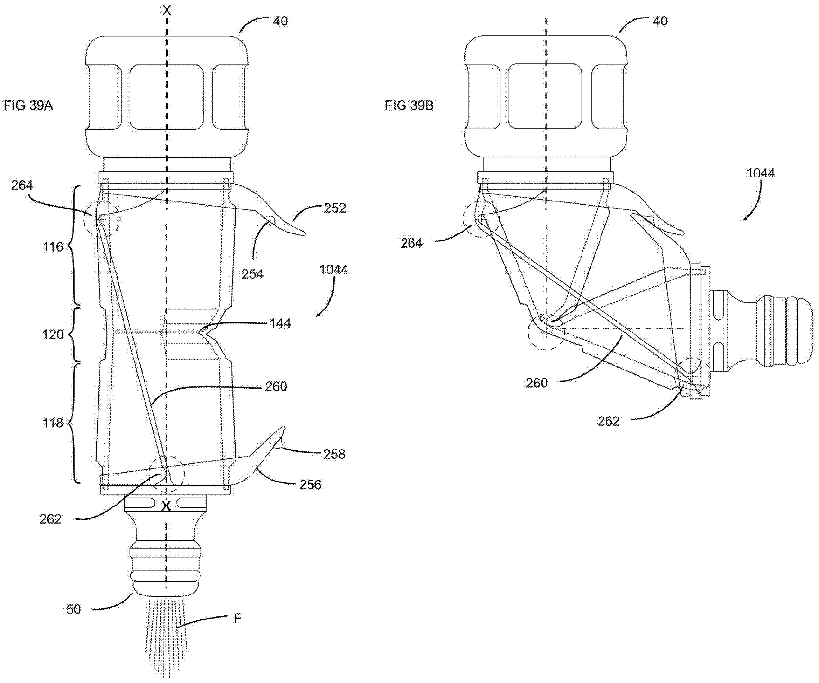

FIGs 39A to 39B show a further embodiment of the invention where an inline closure 1044 is formed in a fluid conduit. The closure 1044 comprises a tubular body having a closure axis X through which a fluid F can pass, and defines hose connectors 40, 50 at both ends.

The closure 1044 comprises a first section 116 and a second section 118 separated by a crease initiation region 120. The first section 116 defines an arm 252 having a female clip part 254. The second section 118 defines an arm 256 having a male clip part 258.

The crease initiation region 120 defines an interior valve seal 144 on the same side as the arms 252, 256. A strap 260 extends from the first section 116 to the second section 118 to maintain the closure 1044 in an open position. The strap 260 is pivoted at both ends 262, 264 by live hinges.

When the user wishes to stop the flow through the pipe, the first and second sections 116, 118 are pivoted relative to each other about the crease initiation region 120 thus developing a kink or hinge in which the valve seal 144 provides a self-sealing effect in the pipe. The closure 1044 is held in the closed position by the interaction of the female and male clip parts 254, 258. The pipe can be opened by disengaging the clip parts 254, 258. The combination of the strap 260 and water pressure will act to open the closure 1044.

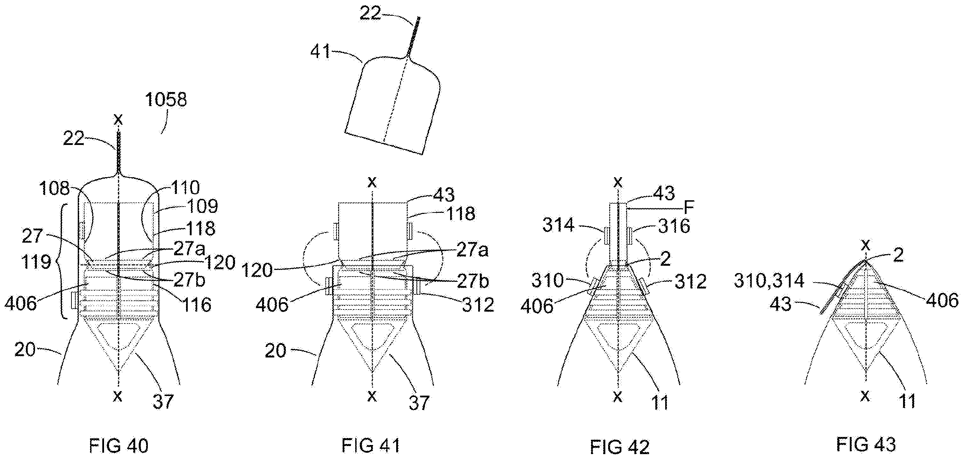

FIGs 40 to 43 show a side cross sectional view of a spout 1058 bonded within the top portion or opening of a pouch 20. FIG 40 shows the spout 1058 to be in an open condition, but sealed within the pouch 20. The spout 1058 has a spout axis X, a first section 116 and a second section 118 spaced therealong joined by a crease initiation region 120. The spout 1058 also has a first sidewall 102 and a second sidewall 104.

The spout 1058 is shown to include a strip valve 27. The strip valve 27 is a strip of material which is formed as an endless loop and bonded to the interior surface of the spout sidewalls within the crease initiation region 120. The strip valve 27 is attached to the interior surface of the spout 1002 at an attachment region 27a, and is free (i.e. not bonded) at a free region 27b below the attachment region 27a. The boundary between the attachment region 27a and the free region 27b initiates a crease 2. The entire strip valve 27 conforms to the curvature of the inner wall when the spout is open to be the same shape as the spout 1058.

The spout 1058 further comprises an internal anti bonding lining 406. The anti-bonding lining 406 is an internal coating of foil in the first section 116, coating both sidewalls 116, 118 which allows the walls to collapse under heat and compression at the moment of being bonded into a pouch. The anti-bonding lining 406 provides a barrier which stops the walls 108, 110 from bonding together whilst allowing the external pouch to be bonded to the internal surface of the spout or closure.

The pouch defines a neck 119 which contains the spout 1058. The neck 119 terminates in a seal 41 having a pull tab 22. The neck 119 and seal 41 are unitary, but separated by a frangible tear seam 43. The frangible tear seam 43 is at the crease initiation region 120.

The pouch 20 defines a first female clip part 310 and a second female clip part 312 on opposite outer surfaces of the neck 119 adjacent the first portion 116. The spout 1058 defines a first male clip part 314 and a second male clip part 316 on opposite sides of the second section 118.

FIG 41 shows a side cross sectional view of the pouch 20 and spout 1058 with the seal 41 removed. Pulling the seal 41 separates the seal 41 from the neck region 118 along the frangible tear seam 43. Thus the contents of the pouch 20 can be poured out from the free end 109. Removal of the seal 41 also exposes the male clip parts 314, 316.

FIG 42 shows a side cross sectional view of the spout 1058 as described in FIGs 40, 41. The spout 1058 is shown to be within the first stages of closing with the sidewalls 108, 110 of the spout meeting at the crease 2 (i.e. within the crease initiation region 120) and throughout the second section 118. In FIG 42, the second section 118 extends vertically upwardly along the spout axis X.

The spout first section 116 starts to "triangulate" as the natural structural stiffness of the spout at the end 37 maintains a gap between the sidewalls of the spout 1058 at the pouch end 37. Because the shape of the first section 116 is "triangulated", the free region 27b of the strip valve 27 comes away from the interior wall of the spout 1058. The free region 27b extends freely and downwardly from the uppermost apex of the formed triangle 51 along the spout axis X.

Turning to FIG 42, a force F is applied to move the second section 118 of the spout 1058 over the first section 116. The first second section 118 is folded along the crease 2 in the direction of the first sidewall 108. As the second section 118 is folded along the crease 2, the free region 27b of the strip valve 27 moves towards, and bears against, the second sidewall 104 of the spout (i.e. the sidewall opposite to the direction in which the second section 118 is folded). In other words, in the closed condition, the valve 27 spans the sidewalls 102, 104 at the crease 2.

FIG 43 shows a side cross sectional view of the spout 1058 in a fully closed condition. The spout second section 118 is shown to be fully folded at the axis crease 2 allowing the male clip part 314 on the second section 118 to engage the female clip part 310 on the outer surface of the pouch 20 adjacent the first section 116. The second section 118 maintains contact via the clip 310, 314. The free region 27b of the strip valve 27 fully bears against the second sidewall 110.

Functionally, any increase in pressure within the pouch 20 (created by e.g. squeezing or inverting the pouch) will act to press the free region 27b of the collapsed strip valve 27 against the sidewall of the spout 1058. This acts to further close the strip valve (the only route through the spout 1058 is through the strip valve 27) and thus the spout 1058 has a "self-sealing" effect.

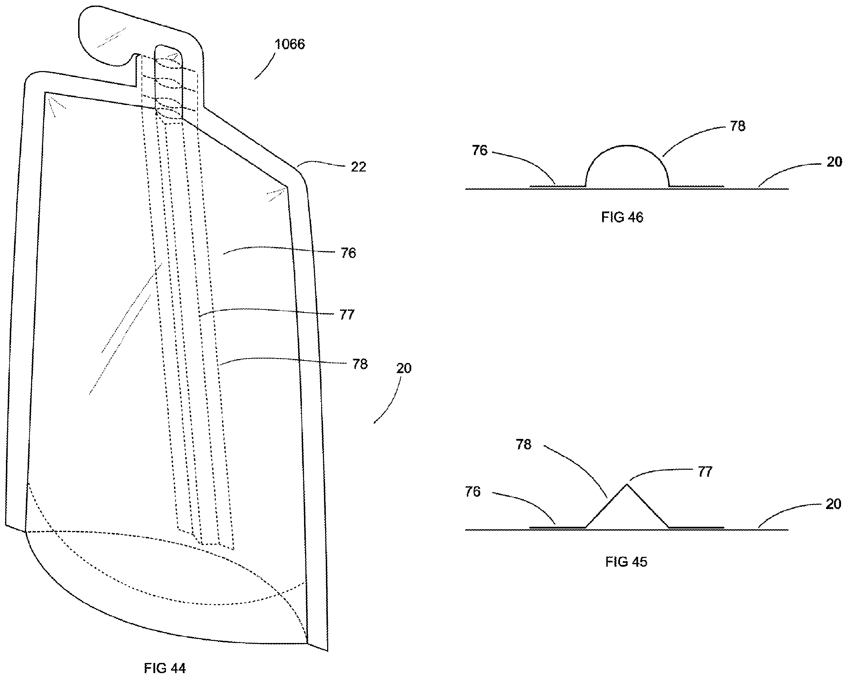

FIGs 44 and 45 show a pouch 20 having an internal straw 78 in fluid communication with a spout 1066 according to the invention. The straw 78 is formed through bonding a strip of concave material to the inner wall of the pouch 20 at contact regions 76. The strip is attached to the interior wall to define a ridge 77 (FIG 45) thus creating a channel through which fluid can be sucked without needing to tip the pouch 20.

FIG 46 is a further example cross section of a straw 78 with a curved cross section.

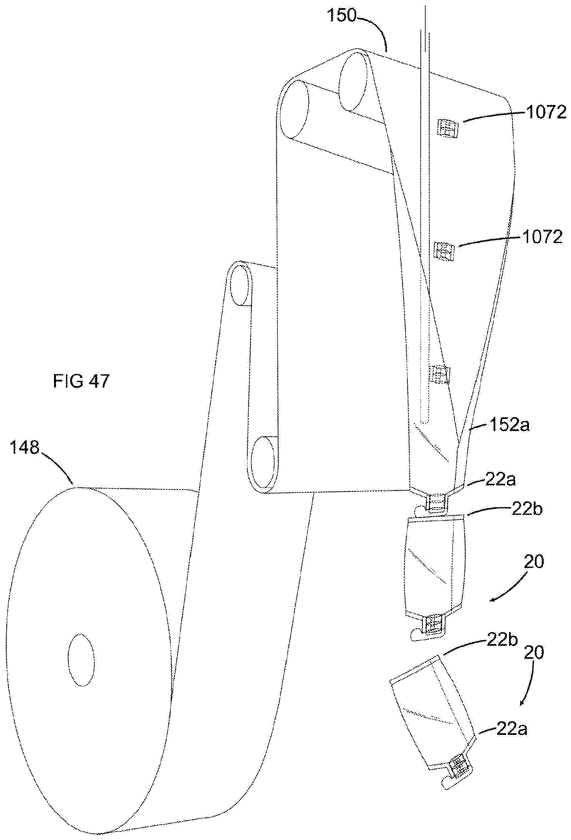

FIG 47 shows a manufacturing process for the formation and assembly of pouches 20 comprising spouts 1072 according to the present invention. A roll 148 of flexible pouch material is provided, and unrolled to form a sheet 150. A plurality of equally spaced spouts 1072 are bonded to one side of the sheet 150 before the sheet is folded around the spouts 1072 to form a tube 152. The pouches 20 are then formed by creating seals 22a, 22b, the former seal 22a being formed over the spout 1072 to include a frangible tear seam and seal as described with reference to the above embodiments.

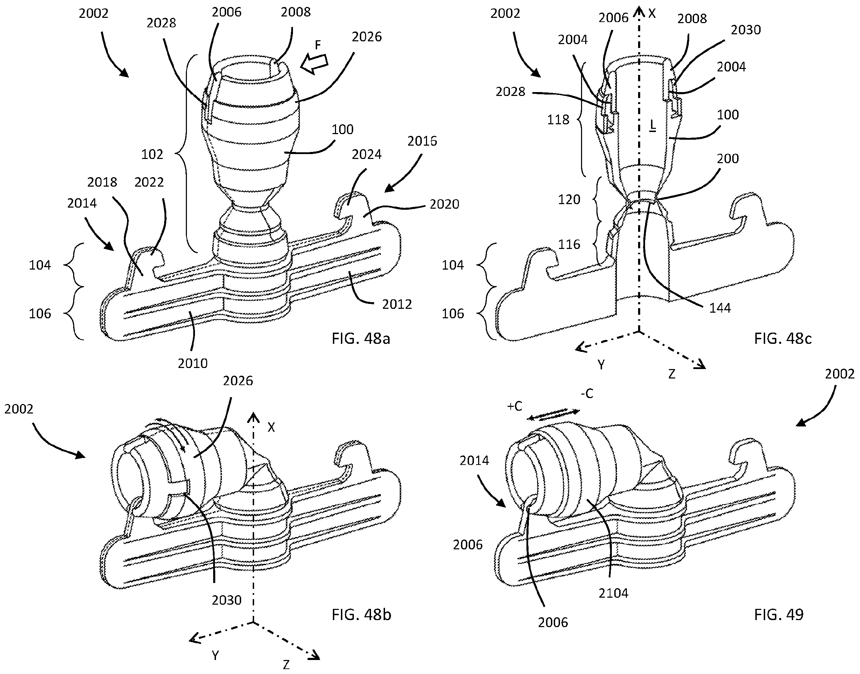

FIGs 48a to 48c are views of a spout 2002 according to the present invention. In FIGs 48a and 48c, the spout 2002 is shown in an open condition, and in FIG 48b in a closed condition.

The spout 2002 comprises a unitary body 100 constructed from an injection moulded plastics material. The body 100 is generally tubular defining a circular lumen L therethrough. The spout 2002 defines a spout axis X therethrough (FIG 48c), which also defines the direction of flow of fluid through the spout 2002 when open.

The spout 2002 also defines two further axes Y and Z perpendicular to the spout axis X (FIG 48c).

The spout 2002 comprises a valve 102, a locking region 104 and an attachment region 106, all of which are defined by the body 100.

Turning to FIG 48c, the valve 102 comprises a first section 116 and a second section 118. The sections 116, 118 are spaced apart in the X direction and joined by a crease initiation region 120.

The first section 116 is adjacent the attachment section 106. The second section 118 is on the opposite side of the crease initiation region to the first section 116. At the free end of the second section 118 (opposite to the crease initiation region 120) there is provided a collar receiving recess 2004 around its circumference. Two diametrically opposed slots 2006, 2008 extend from the free end of the second section 118 to the collar receiving recess 2004.

The crease initiation region 120 has a generally thinner wall thickness than the sections 116, 118 (see Figure 48c). The wall thickness of the crease initiation region tapers (from both the first and second section 116, 118) to become thinnest at a neck 200, where the lumen L is narrowest. In other words, the crease initiation region is shaped as two frustrocones joined at their narrowest ends.

Referring to FIG 48c, at the neck 200 there is provided a valve seal 144 extending in an endless fashion around the inner perimeter of the spout. The valve seal 144 protrudes into the lumen L in both a radial direction and in an axial direction (i.e. towards the second section 118). The seal 144 protrudes at an angle of about 45 degrees. The seal 144 is tapered to a point in cross section and therefore is deformable. The valve seal 144 is therefore cantilevered and extends radially inwardly of the crease initiation region 120 at the neck 200.

The attachment section 106 is comprises a generally tubular part of the body 100 defining an opening to the lumen L in addition to a pair of wings 2010, 2012 extending radially therefrom in the +Y / -Y directions. The attachment section 106 comprises a plurality of weld ribs extending along the wings 2010, 2012.

Extending from the upper surface of each wing 2010, 2012 (i.e. in the X direction) there are provided a pair of spout retention members in the form of hooks 2014, 2016. Each hook 2014, 2016 has an upwardly depending portion 2018, 2020 and a tab 2022, 2024 extending therefrom to form an inverted "L".

The spout 2002 further comprises a collar 2026 which is generally cylindrical in shape. The collar 2026 defines two diametrically opposed slots 2028, 2030 extending from its upper end. The collar 2026 engages the collar receiving recess 2004 in the second section 118. The collar 2026 can be rotated by hand in about the axis X between positions in which the slots 2028, 2030 are aligned with the slots 2006, 2008 and positions in which the slots are misaligned.

In use, the spout 2002 can be moved from the open condition shown in FIG 48a to the closed condition shown in FIG 48b by applying a force F in a direction along the Y axis at the second section 118 whilst holding the attachment section 106 stationary. This creates a leverage and a torque on the valve 102 which tends to deform it. Firstly, because the sidewalls of the crease initiation region 120 are tapered inwardly towards the axis X (due to the shape of the spout 2002 at this point) the vertical load arising from force F (i.e. down the sidewall) Is not supported. As such, it buckles and thus collapse. Also, because the crease initiation region has a reduced second moment of area in the ZY plane due to its thinner wall thickness, smaller cross section (i.e. necked region) and lack of stiffening ribs compared to the first and second sections 116, 118 it collapses. The seal 144 also facilitates collapse by forming a local stress concentration. The crease initiation region flattens, closing the lumen L and resulting in the opposing parts of the seal 144 contacting each other (as with previous embodiments). The collapsed crease initiation region 120 defines a crease axis parallel to the Z axis through the neck 200.

The crease initiation region 120 can be deformed such that the second section 118 meets one of the hooks 2014. At this point, the collar 2026 is in a rotational position such that the slots 2028, 2030 are aligned with the slots 2006, 2008. This allows the hook 2014 to pass through the sidewall of the section 2018. Once in position, the collar 2026 is rotated to take the slots out of alignment. This causes the tab 2022 to catch and retain the collar 2026 and thus lock the second section 118 in position.

When the user wished to open the spout 2002, the collar 2026 is rotated to align the slot 2006 with the hook 2014 thus releasing the second section 118 to the upright position.

In use, the wings 2010, 2012 are sealed within e.g. a pouch leaving the hooks 2014, 2016 exposed for the above method of operation.

Turning to FIG 49, a spout 2102 is identical to the spout 2002 with the exception that instead of a rotatable collar 2026, the spout 2102 is provided with a slidable collar 2104. The collar 2104 can be moved axially between a first position in which it captures the hook 2014 in the slot 2006, and a second position (in direction -C) in which it released the hook 2014 from the slot 2006.

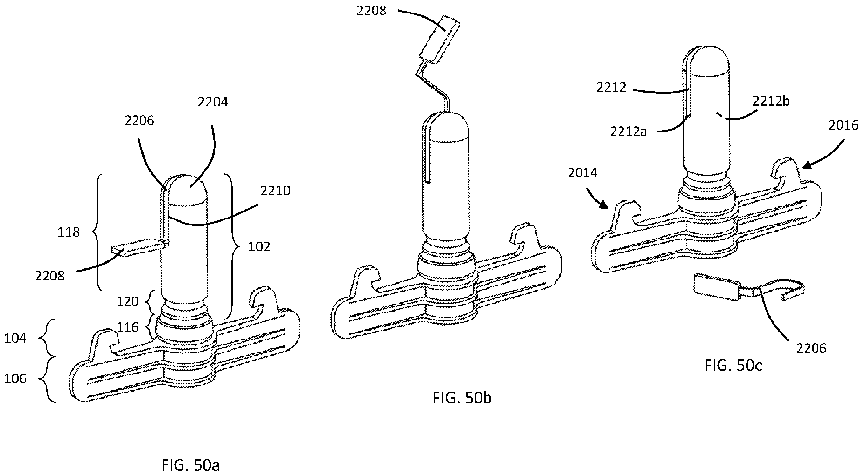

Turning to FIGs 50a to 50c, a spout 2202 is provided being similar to the spouts 2002, 2102. The spout 2202 comprises a valve 102, a locking region 104 and an attachment region 106, all of which are defined by the body 100. The locking region 104 and attachment region 106 are identical to the spouts 2002, 2102.

The valve 102 comprises a first section 116 and a second section 118. The sections 116, 118 are spaced apart in the X direction and joined by a crease initiation region 120. The first section 116 and crease initiation region 120 are identical to the spouts 2002, 2102.

The spout 2202 is shown in FIG 50a in an open, but sealed condition. The second section 118 has a closed free end 2204 opposite the crease initiation region 120. A removable portion 2206 is provided in the free end 2204. The removeable portion 2206 has a pull tab 2208 attached at one end thereof. The removeable portion forms part of the second section 118 when intact (FIG 50a) is connected thereto by a frangible seal line 2210. Therefore pulling on the pull tab 2208 removes the removeable portion 2206 of the second portion leaving a slot 2212 which extends over the free end 2204 of the second portion having a first end 2212a on a first side and a second end 2212b (not visible) on a second side thereof. Effectively, two diametrically opposed (but joined) slots are provided on either side of the second portion 118. These slots are used to retain the spout 2202 in the closed position as with the spouts 2002, 2102.

It will be noted that no collar is provided in the spout 2202, and instead the spout is simply elastically deformed to hook the respective hook 2014, 2016 against the respective end 2212a, 2216b of the slot 2212.

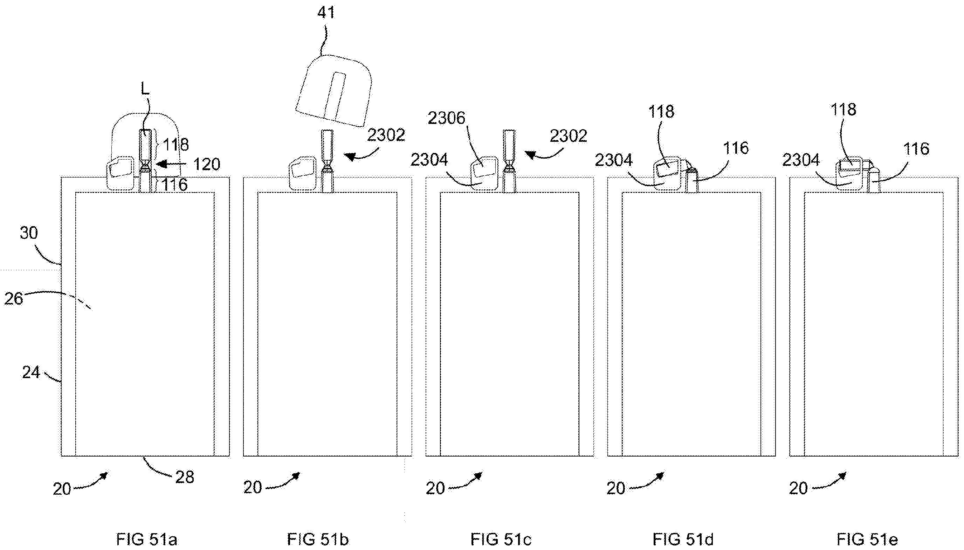

Referring to FIGs 51a to 51e there is shown a spout 2302 assembled with a pouch 20.

The spout 2302 is generally tubular defining a lumen L therethrough. The spout 2302 comprises a first section 116, a second section 118 defining the free, open end and a crease initiation region 120 therebetween. As with previous embodiments, the crease initiation region 120 defines a narrow neck 200 which can seal the spout 2302 when the second section 118 is rotated relative to the first section. The pouch 20 defines an enclosed space for liquid contents. The pouch 20 defines a first side panel 24, a second side panel 26 and a base 28. The panels 24, 26 are attached by a seal 30. The pouch 20 comprises a seal 41 attached to the pouch by a frangible seal line.

The spout 2302 is attached to the pouch 20 such that a lumen L is in fluid communication with the space 22. The spout is attached by sealing the pouch material along the weld ribs of the attachment section. The seal 41 covers and seals the spout 2302 until it is removed (FIG 51b).

Adjacent the spout 2302 there is, attached to the seal 30, a spout retaining tab 2304. The tab 2304 is a flat component having an aperture 2306 therethrough. The tab 2304 is constructed from a stiff plastics or card material. Referring to FIG 51d, when the second section 118 of the spout 2302 is rotated relative to the first section 116, the second section 118 can be inserted into the aperture 2306. The natural resilience of the spout 2302 will try to restore the position of the second section, but instead it will be held in position by the tab 2304. When the user wishes to open the spout 2302, the second section 118 can be retrieved from the aperture 2306.

Although the above embodiments are shown installed in specific pouches, sachets or cartons, they may be installed in any other types of container.

Although it is frequently described that the second section is "folded over" or "folded relative to" the first section, it will be understood that relative rotational movement about the crease is all that is required. In other words, the first sections may be moved with the second section stationary for the invention to operate.

Although many of the above spouts are described as being "lens-shaped", it will be understood that other cross-sectional shapes are possible, including elliptical and circular.

## Claims

1. A tube closure (100) defining a lumen (L) and comprising a valve (102) having:
a first section (116);
a second section (118); and,
a crease initiation region (120) between the first and second sections,
wherein
the crease initiation region (120) formed by a narrowing of the valve and configured to initiate a crease upon relative rotation of the first and section sections; **characterised by**
a protrusion (144) projecting into the lumen (L) from the crease initiation region (102);
in which the valve is movable between:
an open condition in which there is a continuous flow path through lumen (L); and,
a closed condition in which the first section (116) is rotated relative to the second section (118) about a crease formed in the crease initiation region feature to thereby close the lumen in the crease initiation region by abutment of the protrusion with an opposing surface of the valve.

2. A spout (1002) for a flowing consumable material container, the spout comprising a tube closure (100) according to claim 1.

3. A tube closure (100) according to claim 1 or a spout (1002) according to claim 2, in which the protrusion (144) extends at least partially around the interior perimeter of the valve.

4. A tube closure (100) or spout (1002) according to claim 3, in which the protrusion (144) extends in an endless loop around the interior perimeter of the valve.

5. A tube closure (100) or spout (1002) according to claim 3 or 4, in which the protrusion increases in size towards the widest part of the lumen (L).

6. A tube closure (100) or spout (1002) according to claim 5, in which the protrusion (144) comprises a generally crescent-shaped section in plan.

7. A tube closure (100) or spout (1002) according to claim 5 or 6, in which the crease initiation region comprises two opposed sidewalls joined at spaced apart points, in which the protrusion (144) comprises a protrusion portion on each sidewall, in which each protrusion portion increases in size towards the widest part of the lumen (L), and away from the spaced apart points.

8. A tube closure (100) or spout (1002) according to claim 7, in which each protrusion portion is generally crescent-shaped in plan so as to create a lens-shaped cross section upon crease initiation as the protrusion portions come together.

9. A tube closure (100) according to any preceding claim, or a spout (1002) according to any of claims 2 to 8, in which the protrusion (144) defines a flow seal comprising a lip extending into the lumen.

10. A tube closure (100) or spout (1002) according to claim 9, in which the flow seal has a first region attached to the interior sidewall of the valve, and a second, free end, which is configured to bear against the interior surface of the valve in the closed condition.

11. A tube closure (100) according to ay preceding claim or a spout (1002) according to any of claims 2 to 10, comprising a locking arrangement for releasably retaining the valve in the closed condition.

12. A tube closure (100) or spout (1002) according to claim 11, in which the locking arrangement comprises a first part on a first section side of the crease initiation region, and a second part on a second side of the crease initiation region, in which the first and second parts engage to retain the tube closure in the closed condition.

13. A tube closure (100) according to any preceding claim, or a spout (1002) according to any of claims 2 to 12, in which the crease initiation region comprises a local thinning of the sidewall of the valve compared the first and / or second sections.

14. A container comprising a spout according to any of claims 2 to 13.

15. A container according to claim 14 constructed from a flexible sheet material.

## Patentansprüche

1. Rohrverschluss (100), der ein Lumen (L) definiert und ein Ventil (102) umfasst, der aufweist:
einen ersten Teilabschnitt (116);
einen zweiten Teilabschnitt (118); und,
einen Knickinitiationsbereich (120) zwischen dem ersten und dem zweiten Teilabschnitt,
wobei
der Knickinitiationsbereich (120) durch eine Verengung des Ventils gebildet und konfiguriert ist, um einen Knick bei relativer Drehung des ersten und des zweiten Teilabschnitts zu initiieren; charakterisiert durch
einen Vorsprung (144), der von dem Knickinitiationsbereich (102) in das Lumen (L) hineinragt;
wobei das Ventil beweglich ist zwischen:
einem offenen Zustand, in dem ein kontinuierlicher Flussweg durch das Lumen (L) besteht; und,
einem geschlossenen Zustand, in dem der erste Abschnitt (116) relativ zu dem zweiten Abschnitt (118) um einen in dem Knickinitiationsbereich gebildeten Knick gedreht ist, um dadurch das Lumen in dem Knickinitiationsbereich durch Anschlag des Vorsprungs an einer entgegengesetzten Oberfläche des Ventils zu schließen.

2. Ausguss (1002) für einen Behälter für fließendes Verbrauchsmaterial, wobei der Ausguss einen Rohrverschluss (100) nach Anspruch 1 umfasst.

3. Rohrverschluss (100) nach Anspruch 1 oder Ausguss (1002) nach Anspruch 2, wobei sich der Vorsprung (144) zumindest teilweise um den Innenumfang des Ventils erstreckt.

4. Rohrverschluss (100) oder Ausguss (1002) nach Anspruch 3, wobei sich der Vorsprung (144) in einer Endlosschleife um den Innenumfang des Ventils erstreckt.

5. Rohrverschluss (100) oder Ausguss (1002) nach Anspruch 3 oder 4, wobei der Vorsprung zu dem breitesten Teil des Lumens (L) hin größer wird.

6. Rohrverschluss (100) oder Ausguss (1002) nach Anspruch 5, wobei der Vorsprung (144) in Draufsicht einen im Allgemeinen halbmondförmigen Schnitt aufweist.

7. Rohrverschluss (100) oder Ausguss (1002) nach Anspruch 5 oder 6, wobei der Knickinitiationsbereich zwei entgegengesetzte Seitenwände umfasst, die an beabstandeten Punkten verbunden sind, wobei der Vorsprung (144) einen Vorsprungabschnitt auf jeder Seitenwand umfasst, wobei jeder Vorsprungabschnitt zu dem breitesten Teil des Lumens (L) und von den beabstandeten Punkten weg an Größe zunimmt.

8. Rohrverschluss (100) oder Ausguss (1002) nach Anspruch 7, wobei jeder Vorsprungabschnitt in Draufsicht im Allgemeinen halbmondförmig ist, um bei der Knickinititiation einen linsenförmigen Querschnitt zu erzeugen, während die Vorsprungabschnitte zusammenkommen.

9. Rohrverschluss (100) nach einem der vorstehenden Ansprüche oder Ausguss (1002) nach einem der Ansprüche 2 bis 8, wobei der Vorsprung (144) eine Flussdichtung definiert, die eine Lippe, die sich in das Lumen erstreckt, umfasst.

10. Rohrverschluss (100) oder Ausguss (1002) nach Anspruch 9, wobei die Flussdichtung einen ersten Bereich, der an der inneren Seitenwand des Ventils angebracht ist, und ein zweites freies Ende aufweist, das konfiguriert ist, um gegen die innere Oberfläche des Ventils in dem geschlossenen Zustand zu drücken.

11. Rohrverschluss (100) nach einem vorstehenden Ansprüche oder Ausguss (1002) nach einem der Ansprüche 2 bis 10, der eine Verriegelungsanordnung zum lösbaren Halten des Ventils in dem geschlossenen Zustand umfasst.

12. Rohrverschluss (100) oder Ausguss (1002) nach Anspruch 11, wobei die Verriegelungsanordnung einen ersten Teil auf einer ersten Teilabschnittsseite des Knickinitiationsbereichs und einen zweiten Teil auf einer zweiten Seite des Knickinitiationsbereichs umfasst, wobei der erste und der zweite Teil ineinandergreifen, um den Rohrverschluss in dem geschlossenen Zustand zu halten.

13. Rohrverschluss (100) nach einem vorstehenden Anspruch oder Ausguss (1002) nach einem der Ansprüche 2 bis 12, wobei der Knickinitiationsbereich eine lokale Ausdünnung der Seitenwand des Ventils im Vergleich zu dem ersten und/oder dem zweiten Teilabschnitt umfasst.

14. Behälter, der einen Ausguss nach einem der Ansprüche 2 bis 13 umfasst.

15. Behälter nach Anspruch 14, der aus einem flexiblen Plattenmaterial besteht.

## Revendications

1. Fermeture de tube (100) définissant une lumière (L) et comprenant un clapet (102) ayant :
une première section (116) ;
une seconde section (118) ; et,
une région d'amorce de pli (120) entre les première et seconde sections,
dans laquelle
la région d'amorce de pli (120) est formée par un rétrécissement du clapet et conçue pour amorcer un pli lors de la rotation relative des première et secondes sections ; **caractérisée par**
une saillie (144) faisant saillie dans la lumière (L) depuis la région d'amorce de pli (102) ;
dans laquelle le clapet est mobile entre :
un état ouvert dans lequel il existe un trajet d'écoulement continu à travers la lumière (L) ; et,
un état fermé dans lequel la première section (116) est tournée par rapport à la seconde section (118) autour d'un pli formé dans la région d'amorce de pli pour fermer ainsi la lumière dans la région d'amorce de pli par appui de la saillie sur une surface opposée du clapet.

2. Bec (1002) pour récipient de matière consommable fluide, le bec comprenant une fermeture de tube (100) selon la revendication 1.

3. Fermeture de tube (100) selon la revendication 1 ou bec (1002) selon la revendication 2, dans laquelle/lequel la saillie (144) s'étend au moins partiellement autour du périmètre intérieur du clapet.

4. Fermeture de tube (100) ou bec (1002) selon la revendication 3, dans laquelle/lequel la saillie (144) s'étend en une boucle sans fin autour du périmètre intérieur du clapet.

5. Fermeture de tube (100) ou bec (1002) selon la revendication 3 ou 4, dans laquelle/lequel la saillie augmente en taille vers la partie la plus large de la lumière (L).

6. Fermeture de tube (100) ou bec (1002) selon la revendication 5, dans laquelle/lequel la saillie (144) comprend une section généralement en forme de croissant dans le plan.

7. Fermeture de tube (100) ou bec (1002) selon la revendication 5 ou 6, dans laquelle/lequel la région d'amorce de pli comprend deux parois latérales opposées jointes en des points espacés, dans laquelle/lequel la saillie (144) comprend une partie en saillie sur chaque paroi latérale, dans laquelle/lequel chaque partie en saillie augmente en taille en direction de la partie la plus large de la lumière (L), et en s'éloignant des points espacés.

8. Fermeture de tube (100) ou bec (1002) selon la revendication 7, dans laquelle/lequel chaque partie en saillie est généralement en forme de croissant dans le plan de manière à créer une section transversale en forme de lentille lors de l'amorce de pli lorsque les parties en saillie se rejoignent.

9. Fermeture de tube (100) selon l'une quelconque des revendications précédentes, ou bec (1002) selon l'une quelconque des revendications 2 à 8, dans laquelle/lequel la saillie (144) définit un joint d'étanchéité comprenant une lèvre s'étendant dans la lumière.

10. Fermeture de tube (100) ou bec (1002) selon la revendication 9, dans laquelle/lequel le joint d'étanchéité a une première région fixée à la paroi latérale intérieure du clapet, et une seconde extrémité libre, qui est conçue pour s'appuyer contre la surface intérieure du clapet à l'état fermé.

11. Fermeture de tube (100) selon l'une quelconque des revendications précédentes ou bec (1002) selon l'une quelconque des revendications 2 à 10, comprenant un agencement de verrouillage pour retenir de manière libérable le clapet à l'état fermé.

12. Fermeture de tube (100) ou bec (1002) selon la revendication 11, dans laquelle/lequel l'agencement de verrouillage comprend une première partie sur un premier côté de section de la région d'amorce de pli, et une seconde partie sur un second côté de la région d'amorce de pli, dans lequel les première et seconde parties viennent en prise pour maintenir la fermeture de tube à l'état fermé.

13. Fermeture de tube (100) selon l'une quelconque des revendications précédentes, ou bec (1002) selon l'une quelconque des revendications 2 à 12, dans laquelle/lequel la région d'amorce de pli comprend un amincissement local de la paroi latérale du clapet par rapport aux première et/ou seconde sections.

14. Récipient comprenant un bec selon l'une quelconque des revendications 2 à 13.

15. Récipient selon la revendication 14 construit à partir d'un matériau en feuille flexible.
